Europäisches Patentamt

European Patent Office

Office européen des brevets

(19)

(11) **EP 1 731 142 A1**

(12) **EUROPEAN PATENT APPLICATION**

(43) Date of publication:
**13.12.2006 Bulletin 2006/50**

(51) Int Cl.:
*A61K 9/28* (2006.01)     *A61K 9/20* (2006.01)
*A61K 9/48* (2006.01)

(21) Application number: **06252972.2**

(22) Date of filing: **08.06.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **08.06.2005  US 147388**

(71) Applicant: **Dexcel Pharma Technologies Ltd.
91237 Jerusalem (IL)**

(72) Inventors:
• **Penhasi, Adel
  Holon 58671 (IL)**
• **Gomberg, Mila
  Jerusalem 97482 (IL)**
• **Gomberg, Maxim
  Jerusalem 97350 (IL)**

(74) Representative: **Fairbairn, Angus Chisholm
Marks & Clerk
90 Long Acre
London WC2E 9RA (GB)**

(54) **Specific time-delayed burst profile delivery system**

(57)    The invention provides a delivery device for the delayed release of an active agent in the gastrointestinal tract comprising a core, comprising an active agent; a first outer coating, comprising a relatively hydrophobic substantially water insoluble polymer having substantially water insoluble hydrophilic particles embedded therein; and a first inner coating layer, comprising an agent that can cause the dissolution of at least one of the water insoluble components of the outer coating, and optionally a water soluble polymer, such that the insoluble particles in the outer coating, upon absorption of liquid, form channels leading to the inner coating layer, thus enabling the dissolution thereof, whereby the agents contained therein are released to cause the dissolution and/or degradation (destruction) of the outer coating, and the release of the pharmaceutically acceptable active agent from the core of the device.

EP 1 731 142 A1

**Description**

The invention is directed to a device for the oral delivery of active agents in solid dosage forms to specific locations along the gastrointestinal tract and/or delivery after specific lag time by both immediate and sustained release of all or most of the active agent at a predetermined specific location. The active agent delivery system has the capability of loss of integrity in a short space of time thereby allowing the delivery of most to all of the active agent at the location of disintegration.

Delivery to Specific Site/Delayed Release (Time Controlled Delivery)

Specific delivery of drugs to sites in the gastrointestinal tract and/or time controlled delivery of drugs are highly desirable for the treatment of a multitude of conditions. For many drugs an exact delivery to the specific site along the gastrointestinal tract is extremely important. This may be because of extensive degradation of the drug elsewhere in the GI, or because of a narrow absorption window. Thus for the best oral bioavailability, the drug must be protected by the delivery system until it arrives to the right place and then it should be released as fast as possible (so called burst release profile).

It is also desirable to target the drugs to sites in the gastrointestinal tract where the drug could be preferentially absorbed. This may be especially desirable for the delivery of peptide and protein drugs. These latter drugs have proven notoriously difficult to deliver orally due to poor absorption and to degradation by enzymes of the body. Delivery of these drugs to predetermined sites in the GI tract at a high rate of delivery could help alleviate these problems by creating a strong concentration driven gradient for drug absorption while at the same time saturating degradative pathways. Alternately, the drug may be delivered to sites that possess specific carriers to affect the drug delivery.

A delayed release is necessary where the drug should be released after a period of time post administration (lag time). An appropriate example for such a necessity is an early morning release of drugs, e.g., medications taken at night whose actions are required in the early morning hours (chronotherapy).

There are many situations where the active material should be released immediately (after bursting the delaying film coat) in the specific site. These latter situations, therefore, compel designing a delayed fast release system. These systems will be appropriate mainly for drugs that are metabolized to pharmacological active compounds, drugs which have long in-vivo half-lives showing an inherently prolonged duration of action, drugs with a very short in-vivo half-life which require a prohibitively large amount of active ingredient in the dosage form, drugs which are required in large doses for a therapeutic effect, and drugs which are required in very low dose.

Additionally a delayed burst release can also be utilized for enhancing absorption, reducing side effects, increasing bioavailability and decreasing the dose.

Targeting mechanisms for site-specific delivery, such as delivery in the lower gastrointestinal (GI) tract, are based primarily upon the principle of uniform small intestinal transit, pH variability and its effect on eroding film coatings, or chemical or enzymatic conditions in the large intestine that can be exploited to control drug release. There are many oral delayed release technologies, including colon specific drug delivery, which are commercially in use. All of the triggering mechanisms for delayed release, especially colonic delivery, which are widely used amongst the population requiring treatment, are vulnerable to variations based upon time of day, fed state and disease condition. Technologies based on enteric coating are susceptible to pH variations which naturally exist throughout the GI tract of a specific patient as well as existing between different individuals. Technologies based upon exploitation of enzymatic degradation, or other biochemical reactions such as redox potential in the colon, are susceptible to bacterial flora which may vary according to gender, age and race. Accordingly, such systems are not reliable. The concept of osmotic pumps is based upon a tablet or capsule that provides a constant internal pressure as a result of the dissolution of some components, mostly inorganic salts, after penetration of water into the tablet or capsule. The resulting constant pressure inside the core may eventually result in the drug being pushed out at a constant rate. Such technologies are, however, expensive to institute and are designed primarily to provide only a zero order drug release (a constant rate of drug release with time). The technologies that control diffusion from a gel matrix or constant surface area, can result only in a first order or zero order release, respectively, and have no ability to control either site or release profile. Furthermore, the release based on diffusion from a gel matrix may be severely affected by both the viscosity of luminal content as well as the agitation rate of the GI tract.

Several delivery systems were designed with that goal in mind. Following are descriptions of some important ones.

US 4,871,549 10/1989 Ueda, et al. 424/494 discloses a dosage form comprising a core with a drug (or coated with a layer of drug). This is further coated with a synthesized polymer selected from the group consisting of polyvinyl acetate and polyacrylic acid or disintegrating agent selected from the group consisting of hydroxypropylcellulose, sodium starch glycolate and carboxymethylcellulose and overcoated with an insoluble outer membrane (ethyl cellulose). In this system drug release is caused by explosion of the outer membrane occurring after a predefined lag time and mediated by the physical swelling force of the disintegrating agent or the synthesized polymer.

WO 98/32425 7/1998 Busetti et al. A61 K 9/28, US 5,788,987 8/1998 Busetti et al. 424/480US and 5,891,474 4/1999 Busetti et al. 424/490. These patents disclose a formulation for treatment of early morning pathologies. The process for preparation of this formulation includes:

1. Wetting the core containing an active agent and disintegration-enhancing agent with the binder.
2. Coating the core with the particles of swellable polymer to produce a time-specific dosage regulated by a thickness of the swellable polymeric coating layer. The polymeric particles are selected from a group consisting of cellulose derivatives, PVP, PVA, acrylic acid polymer, methacrylic acid copolymers, ethyl acrylate-methyl methacrylate copolymers, natural rubbers, poloxamers, polysaccharides and their mixtures.

This coating delays the release of the drug for about 4 to about 9 hours depending on the thickness of the coating layer. The minimal coat thickness is 50 micrometer ($\mu$m). The core: coating layer thickness ratio ranges between 20:1 and 1:3.

As described in US Patent 5840332 there was developed a new time-controlled release system for the burst release of active material. This system was based on a combination of a new disintegrating core and the novel controllable film coat, consisting of a hydrophobic polymer film coat embedded with non-soluble, but hydrophilic particles. This latter film coat was tailored to undergo splitting under a proper pressure being formed inside the tablet upon the penetration of water. The core was designed to undergo swelling and consequently disintegration to cause simultaneous bursting of the coating film and immediate release of the drug. The delay time (or the lag time), which is the time that it should take until the bursting of the film coat, and thus the release of drug, is adjusted by means of controlling both the thickness of the film coat as well as the weight ratio of said hydrophylic particles in the film coat. By these means, an immediate release of the drug at various locations in the colon will be achieved.

The most serious drawback of this system, however, was found to be the dependence of both film rupture as well as burst release on the weight ratio of excipients/active material. At low ratios no significant burst release can be achieved and in the more extreme cases no rupture of the film coat takes place.

Thus, when the composition of the tablet cannot exert a necessary physical force for the rupture of the film coat and in case of film coated capsules, there is a need for a subtler method in order to achieve the burst release.

In order to obviate this problem, there has been suggested in the prior art, to create a controlled chemical attack on the protective outer coating layer. This attack may come either from within the tablet (or the capsule) or from the outside environment. The following are several publications relating to such systems.

US 5,472,710 12/1995 Klokkers-Bethke, et al. 424/468 discloses a dosage form comprising a core with a drug (or coated with a layer of drug). This is coated with an acid layer containing solid acid (sodium dihydrogenphosphate, citric acid, tartaric acid, succinic acid, fumaric acid), further coated with pH sensitive polymer (cellulose acetate phthalate and other cellulose phthalate derivatives, methacrylic acid copolymers, carboxyethyl methylcellulose) and finally overcoated with a water insoluble outer polymer (ethylcellulose, polyvinylacetate and acrylic and methacrylic acid esters with quaternary ammonium groups) membrane which is permeable to gastric secretions. Optionally the acid layer and the pH sensitive polymer layer may be applied in reverse order or the acid can be included in the drug layer. The acid will protect the pH sensitive polymer layer until its exhaustion by the intestinal medium and then the pH sensitive layer will dissolve and permit the release of the drug through the outer insoluble membrane.

In this system the outer membrane retains its integrity throughout the release, and therefore doesn't burst.

Time controlled drug delivery based on a capsule has been described in the art. WO90/09168 describes the "Pulsincap" system in which a non-soluble capsule body is closed with a hydrogel cap that swells and opens the capsule at predetermined times. While control of the time of drug delivery has been achieved with these systems there are problems with the total delivery of the dose since the capsule remains intact.

T. Ishibashi et al (Journal of Pharmaceutical Sciences 87, 531 (1998)) describes the delivery of drugs from a dissolving capsule whose time of delivery is determined by an outer coating. The outer coating is an acid soluble film while a compatible acid is formulated inside the capsule. When enough water has penetrated the film and dissolved the acid in the capsule, the film is dissolved by the action of the acidic environment. The drug is totally released in a burst fashion. This system requires that the drug being delivered is compatible with the acid in the capsule This system is obviously unsuitable for drugs which can react with acid, and those which are adversely affected by the acidic environment in a dissolved state.

US Patent No. 5,593,697 describes a pharmaceutical implant containing biologically active material, an excipient comprised of at least one water soluble material and at least one water insoluble material, and a polymer film coating adapted to rupture at a predetermined time after implantation. An insoluble outer film controls the access of biological fluids to the inner film which is soluble. The thickness of the outer film controls the time of inner film failure. Upon inner film failure an expanding excipient swells, rupturing the outer film. Systems of this sort lack fine control over the time of rupture since they have only one parameter, that of thickness, to control said time. These systems also require the formulation of the drug with the swelling excipient, which raises questions of compatibility and of bioavailability.

US Patents No. 5,260,069 and 5,472,708 describe a dosage form for delivering drugs, particularly drugs that cannot be released by diffusion. Pellets are comprised of the drug, and a swelling agent that swells when it absorbs water. The pellets are coated by a membrane or coating that is water insoluble but water permeable, containing an insoluble polymer, a water soluble polymer and a permeability reducing agent. The rate of water entry is controlled by the ratio of the three components in the film. Higher proportions of the water soluble polymer weaken the film and make it more permeable while higher proportions of the permeability reducing agent slow down the entry of water. Upon absorption of water the pellets swell and rupture the membrane, thereby releasing the drug. Again in this system one must formulate the drug with the swelling agent in the core of the pellet.

Proceed. Intern. Symp. Control. Rel. Bioact. Mater. 21, (1994), 744 describes what is called the "Chronotropic drug delivery system". Pellets or minitablets of a drug formulation are coated with various thicknesses of a hydrophilic swelling polymer such as hydroxypropylmethylcellulose. When the delivery system is in an aqueous environment the coating swells and slowly dissolves. When it has dissolved, the drug pellets are released and can release their drug load. Control of the time of release is expected to be poor because only one parameter, thickness, is available for said control.

US Patent No. 5260068 describes a capsule that contains pellets. The capsule dissolves in the stomach releasing the pellets. The pellets comprise a drug and an osmotic agent and are coated with a water insoluble but water permeable membrane containing a hydrophobic compound to lower the film permeability to water. The pellets absorb water due to osmotic pressure until the membrane bursts. The relative amount of the hydrophobic material helps to control the rate of water entry and thereby the time of drug release. The drug must be formulated with an osmotic agent and be stable in solutions of high osmolarity.

US Patent 4871549 describes a time controlled exploding system wherein the drug is formulated on a bead in a formulation that comprises the drug and a swelling agent. The swelling agent may be a polymer that swells upon contact with water or may be a gas generating mixture of an organic acid and a carbonate compound. The beads are overlaid with a non-soluble membrane that allows slow water entry. The water allows the swelling agent to burst the coating after a predetermined time lag. This system lacks parameters to control the time of water entry other than coating thickness. It further requires the formulation of the drug with the swelling agent or gas generating agent and its compatibility therewith.

As will be realized, there remains a need for a system that will allow the precise control of the time of drug release, guarantee full drug release and delivery by the complete failure of the protective mechanism, and allow one to formulate a drug with its known compatible excipients, with no need for formulation with excipients that are necessary for the release mechanism. A drug formulated within a capsule with its normal or desired formulation, wherein upon the capsule, on the outer surface, are added two or more coatings that contain all the components to control the time of capsule disintegration and to afford the essentially total disintegration of said capsule, will give the desired effect. The drug is totally separated from the coatings that supply the release mechanisms and therefore no questions of compatibility arise.

According to the present invention, there is now provided a new and modified time-controlled release system for the burst release of an active material. According to the present invention, the drug delivery system has the capability of rapid loss of integrity, thereby allowing the delivery of at least most of the drug load at the location of disintegration.

The mechanism for controlling the site or time of release is a function of the coatings of the solid dosage form thus obviating the need for any special excipients in the formulation for this purpose. However, the release profile of the active agent after bursting of the outer film coat, as opposed to the delivery system which is controlled by the coating layers of the present invention, as described and explained hereinafter, can depend upon the excipients being used in the formulation, the mode of the formulation preparation and the character of the formulation. This feature is important for formulating active agents such as those which should be formulated with a low weight ratio of excipients/drug.

The mechanism of disintegration according to the present invention is affected by an inner film that, when saturated with water, chemically and/or mechanically attacks the outer film. The outer film controls the rate of entry of the water into the inner film thereby controlling the time and site of delivery of the active agent. When the integrity of the outer film has been compromised, the core dissolves, releasing the active agent in either burst fashion or sustained release.

The term "active agent" as used herein is intended to denote any active agent which is suitable and/or desirable for delayed release in the gastro-intestinal tract. Preferred active agents are physiologically acceptable active agents such as vitamins and nutrients, and components known as food additives and especially preferred active agents are pharmaceutically acceptable active agents.

The description hereinafter will be directed primarily to a discussion of delivery of drugs, it being understood that other types of active agents as mentioned above can also be delivered by the devices of the present invention.

More specifically, according to the present invention, there is now provided a delivery device for delayed burst release of an active agent, preferably a physiologically acceptable active agent, and most preferably a pharmaceutically acceptable active agent in the gastrointestinal tract comprising: a) a core comprising a pharmaceutically acceptable active agent; b) a first outer coating comprising a relatively hydrophobic substantially water insoluble polymer having substantially water insoluble hydrophilic particles embedded therein; and c) a first inner coating layer comprising a water soluble polymer and an agent that can cause the dissolution of at least one of said water insoluble components of said outer coating, wherein a plurality of said particles extend from an outer surface to an inner surface of said first outer coating, such that said particles, upon absorption of liquid, form channels leading to said inner coating layer, thus enabling the dissolution thereof, whereby the agents contained therein are released to cause the dissolution of said outer coating, and the burst release of the pharmaceutically acceptable active agent from the core of said device.

Thus, in a first set of preferred embodiments of the present invention, the core containing the drug and the usual excipients are precoated with a first inner film containing an organic acid (citric, tartaric, fumaric, succinic and similar acids) that, when saturated with water, chemically attacks the outer pH-dependent film. The outer film controls the rate of water intake by the inner film thereby controlling the time of the active material release (the lag time). Optionally the core can be precoated with a neutral buffering layer to prevent the contact between the drug and the acid.

As will be realized, this new delivery system comprises a solid dosage form which is loaded with the active material. The solid dosage form is coated with an inner coating that is relatively easily dissolvable in water and contains the agent that will compromise the integrity of the outer coating. This agent can be, for example, an agent that changes the pH of the environment outside the solid dosage form or an agent that reacts with a major component of the outer membrane, rendering it readily soluble in water. The outer coating is one that controls the time and site of drug delivery by controlling the entry of water into the inner coating for a predetermined time period. The outer film coat contains as one of its essential features a major component that is substantially insoluble in water but that can be readily dissolved upon release of the agent in the inner coat. Thus, the outer coating prevents entry of water into the inner coating or into the solid dosage form for a predetermined time period, allows entry of water into the inner layer which dissolves, whereafter, the outer layer is then compromised by an agent from the inner layer. The compromised outer layer undergoes failure allowing quick entry of water into the system. The water readily dissolves the active material (for aqueous soluble active agents) and/or disintegrates the solid dosage form affording total delivery of the drug load at the predetermined site and/or predetermined time. Accordingly, the nature of the agent in the inner coating will be determined by the nature of the outer coating. The drug is therefore delivered to the desired site after a predetermined time without any need to reformulate it with excipients of the delivery system. The release profile of the drug after bursting of the outer film coat, however, can depend upon the excipients being used in the formulation, the mode of the formulation preparation, the character of the formulation and the water solubility of the active material. This feature is important for formulating tablets that may have a high content of the active material resulting in a low weight ratio of excipients/drug or capsules in which the drug load and its formulation are kept totally separate from the components of the delivery system.

Such a system has many advantages. Since drug release and dosage disintegration is not pH related and is not linked to the bacterial flora of the colon, such a system is very flexible and extremely reliable. This technology is unique because the drug release mechanism depends solely upon the presence of GI tract fluids, regardless of the viscosity of luminal content. Depending on the nature of both the core as well as the coating, the release profile can be designed to be either a delayed burst release or delayed rapid slow release. The onset of drug release can be calibrated to precisely target specific segments of the GI tract, including the colon. The lag time (the time from the administration of the drug to the release start) can be readily controlled upon controlling parameters related to both the coating as well as the core. This fact provides for a system that can be easily used for chronotherapy (delivery of a drug in a precise timing according to circadian rhythms for obtaining optimal treatment of diseases) where the peak times of exacerbation occur in the morning, (for example hypertension, asthma, rheumatoid arthritis), or the afternoon & evening (for example osteo-arthritis). Such a system is a versatile technology that can be used for coating different types of solid dosage forms such as granules, microspheres, microparticles, microcapsules, beads, pellets, tablets, caplets, and capsules regardless of the dose. The burst profile can be designed to be independent of the weight ratio of burst controlling agents and/or disintegrants / drug in the core formulation. The system is an appropriate technology for the delivery of any drug substance with different physico-chemical characteristics such as hydrophilic (water soluble), hydrophobic (water insoluble), amorphous, crystalline, hygroscopic (very sensitive to humidity absorption), and lipophilic (highly water insoluble, lipid soluble) drugs. Both the core and the coating of the delivery system are produced using standard pharmaceutical equipment.

A pulsing tablet enabling precise timing for multiple drug releases in a single unit is also possible.

The clinical advantages of such a system are expected to be;

    1. Increased absorption and higher bioavailability than a conventional immediate release or sustained release drug due to the system's ability to release in a burst manner,

    2. Enhanced delivery of poorly bioavailable drugs that would be destroyed in the higher GI tract environment (for example, peptide molecules),

    3. Reduced dose of drug without decrease in therapeutic effect,

    4. Reduced side effects,

5. Reduced drug interactions due to lower receptor concentration of cytochrome P450 isoenzymes,

6. Reduced food effect (the changes occurring in bioavailability of drug when given with food),

7. Improved compliance,

8. Chronotherapy-programmed delayed release of a drug for optimal treatment of disease,

9. Pulse release, which allows multiple dosing in a single dosage form; and

10. Site-specific release for local treatment of diseases

Such a system may also exploit the controlled absorption characteristics of the colon. For example, rapid release of drugs with fast elimination rate in colon would result in an even level of drug plasma concentration due to slow absorption rate.

The technological advantages of such a system are;

1. The drug is protected until its arrival at the site of release;

2. Drug release is not subject to variations in the pH of the gastrointestinal tract;

3. Drug release is not subject to variations of viscosity of lumen contents;

4. The system is not dependent on the agitation rate of the GI tract;

5. The system is not colon flora dependent;

6. The system is not dependent on the nature of the drug;

7. The system offers many parameters for controlling the release profile;

8. The system offers many parameters for controlling the lag time;

9. The system production process is based on standard pharmaceutical equipment;

10. The drug release is not dependent on the weight ratio of excipients/drug; and

11. The system can be utilized for various solid dosage forms such as granules, microspheres, microparticles, tablets, capsules, and pellets.

In one embodiment of the current invention the outer coating that controls the entry of water is a film of a water insoluble polymer, embedded with water insoluble but hydrophilic particles. Such a coating is described in US Patent number 5840332 as a coating for tablets or capsules. Said coating offers many parameters for controlling drug delivery as described in that patent. The identity, weight percent, and size of the particles, and the identity of the hydrophobic polymer, as well as the coating thickness, are all parameters that control the entry of water through such a coating. We have found that certain embodiments of that coating can be used as the outer coating for this invention. According to some embodiments of the present invention, the water insoluble polymer of the outer coating is selected from the group consisting of a dimethylaminoethylacrylate/ethylmethacrylate copolymer, the copolymer being based on acrylic and methacrylic acid esters with a low content of quaternary ammonium groups, wherein the molar ratio of the ammonium groups to the remaining neutral (meth)acrylic acid esters is approximately 1:20, the polymer corresponding to USP/NF "Ammonio Methacrylate Copolymer Type A", an ethylmethacrylate/chlorotrimethylammoniumethyl methacrylate copolymer, the copolymer based on acrylic and methacrylic acid esters with a low content of quaternary ammonium groups wherein the molar ratio of the ammonium groups to the remaining neutral (meth)acrylic acid esters is 1:40, the polymer corresponding to USP/NF "Ammonio Methacrylate Copolymer Type B", a dimethylaminoethylmethacrylate/methylmethacrylate and butylmethacrylate copolymer, a copolymer based on neutral methacrylic acid esters and dimethylaminoethyl methacrylate esters wherein the polymer is cationic in the presence of acids, an ethylacrylate and methylacrylate/ethylmethacrylate and methyl methylacrylate copolymer, the copolymer being a neutral copolymer based on neutral methacrylic acid and acrylic acid esters, ethylcellulose, shellac, zein, and waxes. More preferably, the water insoluble polymer is ethylcellulose or Eudragit E or their combinations thereof.

According to preferred embodiments, the outer coating further comprises hydrophilic water-insoluble particulate matter. The water insoluble particulate matter is more preferably selected from the group consisting of a water insoluble cross-linked polysaccharide, a water insoluble cross-linked protein, a water insoluble cross-linked peptide, water insoluble cross-linked gelatin, water insoluble cross-linked hydrolyzed gelatin, water insoluble cross-linked collagen, water insoluble cross linked polyacrylic acid, water insoluble cross-linked cellulose derivatives, water insoluble cross-linked polyvinyl pyrrolidone, micro crystalline cellulose, insoluble starch, micro crystalline starch and a combination thereof.

According to specific embodiments, the cross-linked polysaccharide is selected from the group consisting of insoluble metal salts or cross-linked derivatives of alginate, pectin, xanthan gum, guar gum, tragacanth gum, and locust bean gum, carrageenan, metal salts thereof, and covalently cross-linked derivatives thereof.

According to specific embodiments, the water insoluble cross-linked cellulose derivatives are selected from the group consisting of cross-linked derivatives of hydroxypropylcellulose (HPC), hydroxypropylmethylcellulose (HPMC), hydroxyethylcellulose, methylcellulose, carboxymethylcellulose, and metal salts of carboxymethylcellulose.

Most preferably, the water insoluble particulate matter is micro-crystalline cellulose or an insoluble metal salt of a polysaccharide or their combinations thereof.

Optionally, the outer coating further comprises a plasticizer. More preferably, the plasticizer includes at least one of dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, butyl and/or glycol esters of fatty acids, refined mineral oils, oleic acid, castor oil, corn oil, camphor, glycerol and sorbitol or a combination thereof.

In one preferred embodiment the hydrophilic non-soluble particles are a crosslinked polysaccharide or an insoluble metal salt of a polysaccharide or microcrystalline cellulose or a mixture of them which is embedded in a polymer that is insoluble at neutral pH but soluble at acid pH. In a most preferred embodiment this polymer is Eudragit E or ethylcellulose or mixture of them.

The inner coat comprises at least one of a binder such as hydroxypropylmethylcellulose or hydroxypropylcellulose, and a rupturing agent that can be at least one organic acid to dissolve the pH sensitive outer polymer, and/or at least one metallic ion chelating agent such as a salt of ethylenediaminetetraacetic acid (EDTA). In a preferred embodiment the organic acid is citric acid. Water enters the outer coating through the filled channels formed by the non-soluble particulate embedded in the outer coating. The inner film dissolves, releasing the organic acid. The release of the organic acid causes the dissolution of the hydrophobic polymer (in the above mentioned most preferred embodiment, Eudragit E) in which the particles are embedded. The outer coating loses its integrity since there is no longer a polymer matrix to hold the particles around the capsule or the core. The gelatin capsule or the core is thus totally exposed and quickly dissolves and/or disintegrates.

In another most preferred embodiment the embedded hydrophilic non soluble particles are a non soluble metal salt of an acidic polysaccharide, most preferably calcium pectinate (CaP) or calcium alginate at more than 50% w/w, while the hydrophobic polymer is ethylcellulose or Eudragit E. Calcium pectinate (CaP) and Eudragit E are the most highly preferred of the embodiments. The inner film consists of a salt of ethylenediaminetetraacetic acid (EDTA) in a water soluble polymer such as hydroxypropylcellulose (HPC) or polyvinylpovidone (PVP). The outer membrane controls the rate of water entry. The EDTA dissolves and then competes for the metal ion with the polysaccharide. The removal of the metal ion from the polysaccharide renders the polysaccharide soluble. Dissolution of the polysaccharide particles (more than 50% of the film) destroys the integrity of the outer film. Water reaches the capsule and/or the core totally resulting in dissolution and/or disintegration of the gelatin capsule or the core, thereby releasing the drug dose in its entirety.

According to specific embodiments the binder of the inner layer is selected from the group consisting of Povidone (PVP: polyvinyl pyrrolidone), polyvinyl alcohol, copolymer of PVP and polyvinyl acetate, HPC (hydroxypropyl cellulose) (more preferably a low molecular weight), HPMC (hydroxypropyl methylcellulose) (more preferably a low molecular weight), carboxy methyl cellulose (more preferably a low molecular weight), ethylcellulose, hydroxyethyl cellulose, gelatin, polyethylene oxide, acacia, dextrin, magnesium aluminum silicate, starch, polyacrylic acid, polyhydroxyethylmethacrylate (PHEMA), polymethacrylates and their copolymers, gum, water soluble gum, polysaccharide, hydroxypropylmethyl cellulose phthalate, polyvinyl acetate phthalate, cellulose acetate phthalate, hydroxypropylmethyl cellulose acetate succinate, poly(methacrylic acid, methyl methacrylate)1:1 and poly(methacrylic acid, ethyl acrylate)1:1, alginic acid, and sodium alginate, and any other pharmaceutically acceptable polymer that dissolves in buffer phosphate pH >5.5 and/or mixtures thereof.

According to specific embodiments of the present invention, the organic acid as the rupturing agent in the inner layer is selected from the group consisting of citric acid, fumaric acid, malic acid, ascorbic acid (Vitamin C), lactic acid, oxalic acid, maleic acid, malonic acid, glutaric acid, adipic acid, pimelic acid, suberic acid, azelaic acid, sebasic acid, tartaric acid, acetic acid, propionic acid, glycolic acid, pyruvic acid, oxalic acid, succinic acid, benzoic acid, cinnamic acid, mandelic acid, methanesulfonic acid, ethanesulfonic acid, p-toluenesulfonic acid, salicylic acid, palmatic acid, and the like, and amino acids selected from the group consisting of aspartic acid and glutamic acid, and any other pharmaceutically acceptable organic acids.

According to preferred embodiments of the present invention, the inner layer further may comprise a chelating agent as the rupturing agent. Preferably, the chelating agent is selected from the group consisting of antioxidants, dipotassium edentate, disodium edentate, edetate calcium disodium, edetic acid, fumaric acid, malic acid, maltol, sodium edentate, trisodium edentate, ethylene diamine tetra acetic acid (EDTA).

Optionally, the chelating agent may be integrated with the organic acid, in the inner layer. Thus the erosion of the outer layer may occur via chemical attacks of both of the outer layer's components, i.e. the hydrophobic water insoluble polymer film as well as the hydrophilic water insoluble particulates.

Optionally and preferably, the inner layer comprises a lubricant. More preferably, the lubricant is selected from the group consisting of stearate salts; stearic acid, corola oil, glyceryl palmitostearate, hydrogenated vegetable oil, magnesium oxide, mineral oil, poloxamer, polyethylene glycol, polyvinyl alcohol, sodium benzoate, talc, sodium stearyl fumarate, compritol (glycerol behenate), and sodium lauryl sulfate (SLS) or a combination thereof. Most preferably, the lubricant is talc.

According to some embodiments the outer coating is further coated with an enteric coating. Accordingly, The

**EP 1 731 142 A1**

enteric coating is more preferably selected from the group consisting of hydroxypropylmethyl cellulose phthalate, polyvinyl acetate phthalate, cellulose acetate phthalate, hydroxypropylmethyl cellulose acetate succinate, poly(methacrylic acid, methyl methacrylate)1:1, poly(methacrylic acid, ethyl acrylate)1:1, alginic acid, and sodium alginate.

The outer enteric coating may further comprise a plasticizer. The plasticizer preferably includes at least one of dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, butyl and/or glycol esters of fatty acids, refined mineral oils, oleic acid, castor oil, corn oil, camphor, glycerol and sorbitol or a combination thereof.

According to another embodiment, a further water soluble inner neutral buffering layer coating separates between the inner coating and core. Such a layer may be free of any dissolution agent, and can be used in the case that the core includes acid-sensitive active material. Such a layer may be selected from the group consisting of Povidone (PVP: polyvinyl pyrrolidone), polyvinyl alcohol, copolymer of PVP and polyvinyl acetate, HPC (hydroxypropyl cellulose) (more preferably a low molecular weight), HPMC (hydroxypropyl methylcellulose) (more preferably a low molecular weight), carboxy methyl cellulose (more preferably a low molecular weight), ethylcellulose, hydroxyethyl cellulose, gelatin, polyethylene oxide, acacia, dextrin, magnesium aluminum silicate, starch, polyacrylic acid, polyhydroxyethylmethacrylate (PHEMA), polymethacrylates and their copolymers, gum, water soluble gum, polysaccharide, hydroxypropylmethyl cellulose phthalate, polyvinyl acetate phthalate, cellulose acetate phthalate, hydroxypropylmethyl cellulose acetate succinate, poly(methacrylic acid, methyl methacrylate)1:1 and poly(methacrylic acid, ethyl acrylate)1:1, alginic acid, and sodium alginate, and any other pharmaceutically acceptable polymer that dissolves in buffer phosphate pH >5.5 and/or mixtures thereof.

According to some embodiments, the core or capsule may comprise at least one of an absorption enhancer, a binder, a disintegrant, a hardness enhancing agent, and another excipient.

Accordingly, a delivery system is described that allows a drug, including a sensitive drug, to be formulated in its regular manner with excipients that are known to be compatible with said drug and to impart to the drug any desired improved properties such as stability or absorption enhancement, and allows that drug to be packed in a capsule, which capsule can be coated to allow precise delivery under precise time control. Thus, the drug delivery system of the invention provides a method for the oral delivery of a drug or other bioactive moiety to a patient in need of said agent wherever it is necessary in the gastrointestinal tract. The invention is useful for the precise release of the drug at the site of action to fight local diseases or at a preferred site of absorption to allow enhanced absorption of the pharmaceutical compound or reduced side effects.

List of drugs for which such a system may be useful:

Active agents that can be incorporated in delivery device of the present invention include any bioactive agent. Without limiting the scope of the present invention, suitable drugs include those drugs presented in current edition of Goodman and Gilman's "The Pharmacological Basis of Therapeutics" or the current edition of The Merck Index. Both volumes list drugs suitable for numerous types of therapeutic applications, including drugs in the following categories: drugs acting at synaptic and neuroeffector junctional sites, drugs acting on the central nervous system, drugs that influence inflammatory responses, drugs that affect the composition of body fluids, drugs affecting renal function and electrolyte metabolism, cardiovascular drugs, drugs affecting gastrointestinal function, drugs affecting uterine motitity, chemotherapeutic agents for parasitic infections, chemotherapeutic agents for microbial diseases, antineoplastic agents, immunosuppressive agents, drugs affecting the blood and blood-forming organs, hormones and hormone antagonists, dermatological agents, heavy metal antagonists, vitamins and nutrients, vaccines, oligonucleotides and gene therapies.

The active agent that can be delivered by the novel device of this invention, includes inorganic and organic compounds without limitation, including drugs that act on the peripheral nerves, adrenergic receptors, cholinergic receptors, nervous system, skeletal muscles, cardiovascular system, smooth muscles, blood circulatory system, synaptic sites, neuroeffector junctional sites, endocrine and hormone systems, immunological system, reproductive system, skeletal systems, autocoid systems, alimentary and excretory systems, inhibitory and histamine systems, and those materials that act on the central nervous system such as hypnotics and sedatives.

Classes of active agents that can be used in the present invention include anti-hypertensives, immunosuppressants, anti-inflammatories, diuretics, antiepileptics, cholesterol lowering drugs, hormonals hypoglycemics, antiviral drugs, nasal decongestants, antimicrobials, anti-arthritics, analgesics, anti-cancer drugs, anti-parasitics, proteins, peptides, CNS stimulants, CNS depressants, 5 HT inhibitors, anti-schizophrenics, anti-Alzheimer drugs, anti-psoriatics, steroidals, oligonucleotides, anti-ulcer drugs, proton pump inhibitors, anti-asthmatics, thrombolytics and vitamins.

The present invention is particularly useful for the administration of polypeptides, including proteins, such as, but not limited to, therapeutic agents, nutritional products, steroids, hormones, growth hormone (GH), growth hormone releasing hormone (GHRH), epithelial growth factor, vascular endothelial growth and permeability factor (VEGPF), nerve growth factor, cytokines, interleukins, interferons, GMCSF, hormone-like products, neurological factor, neurotropic factor,

8

neurotransmitter, neuromodulator, enzyme, antibody, peptide, proteic fragment, vaccine, adjuvant, an antigene, immune stimulating or inhibiting factor, heomatopoietic factor, anti-cancer product, anti-inflammatory agent, anti-parasitic compound, anti-microbial agent, cell proliferation inhibitor or activator, cell differentiating factor, blood coagulation factor, immunoglobulin, anti-angiogenic product, negative selective markers or "suicide" agent, toxic compound, anti-angiogenic agent, and the like, and structurally similar bioactive equivalents thereof.

Specific examples of peptide drugs include, but are not limited to, zestril, prinivil, zoladex, calcitonin, sandostatin, lupron, accolade, glucagen, integrilin and hirudin. These drugs have been indicated for hypertension, prostate hypertophy or cancer, osteoporosis, aromegally, asthma, hypoglycemia and anti-coagulation.

Any active agent may be used according to this invention by the person skilled in the art, for example the following agents may be used:

Abacavir, Abciximab, Acarbose, Acebutalol, Acenocourmarol, Acetazolamide, Acetocholine, Acetretin, Acetylcysteine, Acetylsalicylic acid, Acipimox, Acyclovir, Adapalene, Adefovir, Adnosine, Agalsidase alfa, Albendazole, Aldesleukin, Alefacept, Alemtuzumab, Alendronate, Alfacalcidol, Alfentanil, Alfuzosin, Aglucerase, Allopurinol, Alprazolam, Alprostadil, Aluminium Chlorhydrate, Alumin Hydrox, Amantadine, Amethocaine, Amifostine, Amiloride, Aminacrine, Aminophylline, Amino salycilic acid, Amiodarone, Amisulpride, Amitriptyline, Amlodipine Besylate, Amlodipine Maleate, Amoxycillin, Amphotericin B, Ampicillin, Amprenavir, Anagrelide Hcl, Anastrozole, Androgel, Antipyrine, Apomorphine Hcl, Apraclonidine, Aprenavir, Aprotinin, Ascorbic Acid, Aspirin, Atavaquone, Atenolol, Atorvastatin, Atosiban, Atracurium Besylate, Atropine sulphate, Auranofin, Aurothioglucose, Azathiaprine, Azelaic acid, Azelastine, Azithromycin, Aztreonam,

Bacitracin, Baclofen, Barium sulfate, Basiliximab, Becaplermin, Beclomathasone, Benazepril, Benoxinate Hcl, Benserazide, Benzalkonium Hcl, Benzanthine Penicllin G, Benzethonium chloride, Benzhexol Hcl, Benzocaine, Benzoic acid, Benzoxonium chlor, Benzoyl peroxide, Benzydamine Hcl, Benzyl Benzoate, Benzyl Penicillin sod, Benzyl peroxide, Betaine, Betahistine, Betamethasone, Betaxolol, Bevacizumab, Bezafibrate, Bicalutamide, Bifonazole, Bimatoprost, Biperiden Hcl, Bisacodyl, Bismuth Oxychloride, Bismuth subgalic, Bismuth subsalicylate, Bismuth subnitrate, Bisoprolol Fumarate, Bivalirudin, Bleomycin Sulph., Boric acid, Bortezomib, Bosentan, Botulinum Toxin, Bretylium Tosylate, Brimonidine Tart, Brinzolamide, Bromazepam, Bromohexine Hcl, Bromocriptine, Brotizolam, Buclizine Hcl, Budesonide, Bupivacaine, Buprenorphine, Bupropion, Buserelin, Buspirone Hcl, Butenafine Hcl, Busulfan, Butandiol,

Cabergoline, Caffeine, Calamine, Calcipotriol, Calcitonin, Calcitriol, Calcium carbonate, Calcium Chlor, Calc. Folinate, Calcium Glubionate, Calcium Gluceptate, Calcium Heparin, Candesartan Cilexetil, Capecitabine, Capromab Pendetide, Capsaicin, Captopril, Carbachol, Carbamazepine, Carbamide, Carbaryl, Carbidopa, Carbocysteine, Carbon, Carbophos, Carboplatin, Carvedilol, Caspofungin, Cefaclor, Cefadroxil, Cefazolin, Cefepime Hcl, Cefixime, Cefonicid, Cefotaxime, Ceftazidime, Ceftibuten, Ceftriaxone, Cefuroxime, (as axetile), Cefuroxime Sod., Celecoxib, Cepalin, Cephalexin, Cephalothin, Cetalkonium Chlor, Cetirizine, Cetrimide, Cetrimonium Brom, Cetrorelix, Cetylpyridinium Chlor, Charcoal, Chenic acid, Chlorambucil, Chloramphenicol, Chlorbutol, Chlordiazepoxide, Chlorhexidine Gluc., Chlorimipramine Hcl, Chloroprocaine, Chlorquinadol, Chloroquine Phos. BP, Chloroxylenol, Chlorpheniramine Maleate, Chlorpromazine, Chlorpropamide, Chlorthalidone, Cholecalcferol, Cholestyramine, Choline Salicylate, Choriogonadotropin, Ciclopiroxolamine, Cilastatin, Cilazapril, Cimetidine, cinnarizine, Ciprofibrate, Ciprofloxacin, Cisapride, Cisplatin, Citric Acid, Citalopram, Cladribine, Clarithromycin, Clavulanic acid, Clidinium Brom., Clindamycin Hcl, Clindamycin Phos., Cliquinol, Clobazam, Clobetasol Propionate, Clobetasone, Clomiphene citrate, Clomipramine Hcl, Clonazepam, Clonidine Hcl, Clopidogrel, Clorazepate Dipotassium, Clostridium Botulinium, Clotiapine, Clotrimazole, Cloxacillin Sod. Clozapine Coal Tar, Codeine Phos., Colchicine, Colestipol Hcl, Colistimetate Sod., Colloidal Oatmeal, Copolymer-1, Cortisone Acetate, Cromolyn Sod., Crotamiton, Cyanocobalamin, Cyclophosphamide, Cycloserine, Cyclosporine, Cyproterone, Cystosine Arabinoside, Cytarabine.

D-Trp-LHRH, Dacarbazine, Daclizumab, Dalfopristin, Danazol, Dantrolene Sod., Dapsone, Daptomycin, Darbepoetin Alfa, Daunorubicine, Demethyl Chlortetracycline, Deferoxamine Mesylate, Desflurane, Desipramine Hcl, Desloratadine, Desmopressin, Desogestrel, Desonide, Desoximetasone, Dexachlorpheniramine, Dexamethasone, Dexmedetomidine, Dexpanthenol, Dexrazoxane, Dextran, Dextromethorphan Hbr, Diacerein, Diazepam, Dibenzepin, Diclofenac Diethylamine, Diclofenac Sod., Diclofenac Potassium, Dicyclomine Hcl, Didanosine, Diflucortolone, Digoxin, Dihydroergotoxine, Diltiazem, Dimenhydrinate, Dimercaprol BP, Dimethicone, Dimethyl Ether, Dimetindenum, Dinoprostone, Dinoprost Tromethamine, Diphenhydramine Hcl, Dipivefrin, Dipyridamole, Dipyrone, Disodium Clodronate, Disopyramide Phos., Dithranol, Dobutamine Hcl, Docetaxel, Docosanol, Domperidone, Donepezil, Dopamine, Dornase Alpha, Dorzolamide Hcl, Doxazosine, Doxepin, Doxorubicin, Doxycycline, Doxylamine Succinate, Dronabinol, Drospirenone, Drotrecogin Alfa, Dudasteride, Dydrogesterone, Dyphylline,

Econazole Nitr., Efavirenz, Eletriptan Hydrobrom. Emedastine, Enalapril Maleate, Enflurane, Enfuvirtide, Enoxaparin, Entacapone, Ephedrine, Epinephrine, Epirubicin Hcl, Epoeitin Beta, Epoprostenol, Eptifibatide, Ergotamine Tart, Ertapenem, Erythromycin, Erythropoietin, Escitalopram, Esdepallethrin, Esmolol Hcl, Esomeprazole, Estradiol, Estramustine, Estriol, Etanercept, Etambutol, Ethanolamine Oleate, Ethinylestradiol, Ethyl Chloride, Etidronate Disodium, Etodolac, Etomidate, Etoposide VP, Estoricoxib, Eugenol, Exemestane,

Famciclovir, Famotidine, Felodipine, Fentanyl, Ferric (III) Polymaltose Complex, Ferric (III) Sucrose Complex, Ferrous Calc. Citr, Ferrous Fumarate, Ferrous Gluconate, Ferrous Sulph, Fexofenadine Hcl, Finasteride, Flecainide Acet., Flucinamide, Fluconazole, Fludarabine Phos., Flumazenil, Flumethasone, Flunitrazepam, Fluocinolone, Fluocortolone, Fluorometholone, Fluorouracil, Fluoxetine Hcl, Flupenthixol, Fluphenazine, Flutamide, Fluticasone Prop, Fluvastatin Sod, Fluvoxamine Maleate, Folic acid, Follitropin, Fomepizole, Formoterol Fumarate, Fosamprenavir, Foscarnet Trisodium, Fosfomycin, Fotemustine, Furosemide, Fusidic acid,

Gabapentin, Gadobenic acid, Gadodiamide, Gadolimium, Galantamine, Ganciclovir, Ganirelix, Gefitinib, Gemcitabine, Gentamicin, Gestodene, Glibenclamide, Glimepiride, Glipizide, Glucagon, Glycerine, Glycerophosphate, Gonaderalin, Goserelin, Gradoteric acid, Gramicidin, Granisetron, Griseofulvin, Guaiphenesin,

Haloperidol, Halothane, Heparin, Hexamine Hipp, Human Chorionic Gonadotrophin (HCG), Human Post Menopausal Gonadotrophin, Hyaluronidase, Hydrochlorothiazide, Hydrocortisone, Hydrogen Perox, Hydromorphone, Hydroquinone, Hydroxychloroquine Sulph., Hydroxyethyl Starch, Hydroxyprogesterone, Hydroxypropylmethyl Cellulose, Hydroxyzine Hcl, Hydrogen Perox, Hylan,

Ibuprofen, Ichthyol, Idarubicin, Idoxuridine, Ifosfamide, Iloprost, Imatinib, Imipenem, Imipramine Hcl, Imiquimod, Indapamide, Indinavir, Indocyanine Green, Indomethacin, Infliximab, Inocor Lactate, Interferon, Interferon Beta, Iobitridol, Iodine, Iodihanol, Iohexol, Iomeprol, Iopamidol, Iopromide, Ioversol, Ioxitalamate, Ipratropium Brom., Irbesartan, Irinotecan Hcl Trihyd., Iron, Iron (as Ferucarbotran), Isoconazole Nitrate, Isoniazide, Isoflurane, Isopropanolol, Isoproternol Hcl, Isosorbide Dinitrate, Isosorbide Mononitrate, Isothipendyl, Isotretinoin, Itraconazole,

Kaolin, Ketamine Hcl, Ketoconazole, Ketoprofen, Ketorolac Tromethamine, Ketotifen,

L-Asparginase, L-Carnitine, Labetalol Hcl, Lactic acid, Lactitol Monohydrate, Lactulose, Lamivudine, Lamotrigine, Lanreotide, Lansoprazole, Latanoprost, Leflunomide, Lenograstin, Lercanidipine Hcl, Letrozole, Leuprolide Acetate, Leucovorin Calcium, Levamisole, Levobunolol Hcl, Levocobastine, Levodopa, Levofloxacin, Levomepromazine, Levonorgestrel, Lidocaine Hcl, Lignocain Hcl, Linezolid, Lindane, Lisinopril, Lithium Carbonate, Lodoxamide Tromethamine, Lomefloxacin Hcl, Loperamide, Lopinavir, Loratadine, Lorazepam, Lornoxicam, Losartan Potass., Mag. Salts, Malathion, Maprotiline Hcl, Mebendazole, Mebeverine Hcl, Mebhydrolin, Mechlorethamine Hcl, Medroxyprogesterone Acetate, Mefloquine, Meglumine, Melissa, Melphalan, Mematine Hcl, Menotrophin, Mepivacaine, Meprobamate, Mepyramine Maleate, Mercaptopurine, Meropenem, Mesna, Methoxsalen, Metolazone, Mestrolone, Metformin, Methadone, methimazole, Methoexitol, Methotrexate, Methsuximide, Methyldopa, Methylergometrine Maleate, Methylphenidate Hcl, Methylprednisolone, Methylsalicylate, Metoclopramide, Metopimazine, Metoprolol Tart, Metronidazole, Mezlocillin, Mianserin Hcl, Miconazole, Midazolam, Midodrine, Mifepristone, Miglustat, Milnacipran, Milrinone, Miltefosine, Minocycline Hcl, Minoxidil, Mirtazapine, Misoprostol, Mitomycin, Mitoxantrone Hcl, Mizolastine, Moclobemide, Modafinil, Moexipril, Mometazone Furate, Montelukast Sod., Morphine Hcl., Morphine Sulph, Morpholin Salicyl, Moxifloxacin, Mupirocin, Muromonab CD3, Mycophenolate Mofetil, Mycophenolic,

Nabumetone, N-Acetylcysteine, Nadroparin, Nafarelin (as Acetate), Naftifine, Nalbuphine Hcl, Naloxone, Naltrexone, Naphazoline, Naproxen, Naratriptan Hcl, Nedocromil Sod., Nefopam, Nelfinavir, Neoepinephrine Bitart., Neomycin Sulph., Neostigmine, Nesiritide, Nevirapine, Niclosamide, Nicotine, Nicotinic Acid, Nicoumalone, Nifedipine, Nimesulide, Nimodipine, Nitrazepam, Nitrofurantoin, Nitrofurazone, Nitroglycerine, Nonoxynol 9, Norelgestromin, Norepinephrine, Norethisterone Acet., Norfloxacin, Norgestimate, Norgestrel, Nortryptyline Hcl, Nystatin,

Ocreotide, Ofloxacin,Olanzapine, Olmesartan, Olsalazine Sod., Omeprazole, Ondansetron, Opipramol, Orlistat, Orphenadrine, Oseltamivir, Oxandrolone, Oxazepam, Oxcarbazepine, Oxerutin, Oxolamine Citr., Oxomemazine, Oxybutynin, Oxycodon Hcl, Oxycodone Terephthalate, Oxymetazoline Hcl, Oxytetracycline, Oxytocin

Paclitaxel, Palivizumab, Pamidronate, Pancreatin, Pancrelipase, Pancuronium Brom, Panthenol, Pantoprazole, Papaverine, Paracetamol, Paradichlorobenzene, Paromomycin, Paroxetine, Peginterferon Alfa-2A, Pemetrexed, Pemoline, Penfluridol, Penicillin, Pentazocine, Pentoxifylline, Peppermint oil, Pepsin, Pergolide Mesylate, Permethrin, Perphenazine, Pethidine Hcl, Phenazone, Phenazopyridine, Pheniramine Maleate, Phenobarbital Sod., Phenobarbitone, Phenolphthalein, Phenothrin, Phenoxybenzamine Hcl, Phenoxymethylpenicillin, Phentermine, Phentolamin Methansulphonic, Phenylephrine, Phenylpropanolamine, Phenyltoloxamine, Phenytoin Sod., Phospholipid, Phytomenadione, Pilocarpine, Pimacrolimus, Pimozide, Pindolol, piperacillin Sod., Piperonyl Butoxide, Piroxicam, Podophyllotoxin, Polidocanol, Polifeprosan, Polymyxin Sulph., Polystyrene Sulphonate, Polyvidone, Polyvinyl, Porfimer Sod., Potassium Chlor, Potassium Citr., Potassium Gluconate, Potassium Guaiacolsulphonate Cod-Guaiacol, Povidone Iodine, Pramoxine Hcl, Pravastatin Sod., Praziquantel, Prazosin Hcl., Prednisolone, Prednisone, Premethrin, Prilocaine, Primidone, Pristinamycine, Procaine Hcl, Procyclidine, Progesterone, Proguanil, Promethazine, Propafenone Hcl, Propericiazine, Propofol, Propoxyphene Hcl, Propranolol, Propylthioracil, Protamine Sulf., Protirelin Thyroid, Pseudoephedrine, Psyllium Hydrophyl Mucilloid, Pyrantel Pamoate, Pyrethrin, Pyridostigmine, Pyrilamine Maleate, Pyrimethamine, Pyrithion Zinc,

Quetiapine (as fumarate), Quinagolide Hcl, Quinidine, Quinupristin,

Raloxifene, Ramipril, Ranitidine, Reboxetine, Remifentanil Hcl, Repaglinide, Ribavirin, Rifabutin, Rifampicin, Riluzole, Rimantadine, Risedronate Sod., Risperidone, Ritodrine, Ritonavir, Rituximab, Rivastigmine Hydrogen Tart, Rizatriptan, Ropinirole, Ropivacaine, Roruronium Brom., Rosiglitazone, Rosuvastatin, Roxithromycin, Rutin, Salbutamol, Salmeterol, Salmon Calcitonin (Synthet.), Salicylic acid, Saquinavir Mesylate, Scopolamine Hcl, Selegiline, Selenium, Senna, Sertraline Hcl, Sevelamer Hcl, Sevoflurane, Sibutramine, Sildenafil, Silver Sulfadiazine, Simethicone, Simvastatin, Sirolimus, Sod. Bicarb., Sod. Biphos., Sod. Cellular Phos., Sod. Chloride, Sod. CIT., Sod. Cromoglycate, Sod. Fluoride, Sod. Fluoroscein, Sod. Fusidate, Sod. Hyluronate, Sod. Nitroprusside, Sod. Phos., Sod. Polystyrene Sulph., Sod. Stibogluconate, Sod. Sulfacetamide, Sod. Valproate, Somatotrophin, Sotalol, Spiramycine, Spironolactone, Stavudine, Streptokinase, Streptozocin, Succinyl-Choline Chlor, Sucralfate, Sulbactam, Sulfacetamide, Sulfadoxine, Sulfisoxazole, Sulphamethoxazole, Sulphasalazine, Sulpiride, Sulthiame, Sumatriptan,

Tacalcitol Monohydrate, Tacrolimus, Tadalafil, Tamioxifen, Tamsulosin Hcl, Tartaric Acid, Tazarotene, Tazobactam, Tegaserod, Teicoplanin, Temolozamide, Temoporfin, Tenofovir Disoprovil Fumarate, Terazosin, Terbinafine, Terbutaline Sulph., Teriparatide, Testosterone, Testosterone Enanthate, Testosterone Prop., Testosterone Undecanoate, Tetracaine Hcl, Tetracosactide, Tetracycline Hcl., Tetrahydrazoline, Thalidomide, Theophylline Anhyd., Theophylline Sod. Glyc., Thioguanine, Thiopental Sod., Thioridazine, Thymol, Thyrotropin, Thyrotropin Alpha, Thyroxine, Tiapride, Tibolone, Ticarcillin Sod., Ticlopidine Hcl., Timolol Maleate, Tinidazole, Tirofiban Hcl Monohydrate, Tobramycin, Tolbutamide, Tolnaftate, Tolterodine Tartrate, Topiramate, Topotecan Hcl., Tacrolimus (as Monohydrate), Tramadol Hcl.,Tranexamic Acd., Trastuzumab, Travaprost, Trazarotene, Trazodone, Treprostinil, Tretinoin (See Vit. A), Triamcinolone, Triazolam, Tribenoside, Triclofos Sod., Triclosan, Trihexyphenidyl, Trimethoprim, Trimipramine, Triprolidine, Triptoreline, Trisod. Cit, Trolamine, Tromantadine Hcl, Tropicamide, Troxerutin, Tubocurarine, Tyrothricin,

Undecylenate, Urea Hydrogen Peroxide, Urofollitropin, Urokinase, Ursodeoxycholic acid,

Valaciclovir, Valerian, Valganciclovir, Valproic acid, Valrubigin, Valsartan, Vancomycin Hcl, Vardenafil, Vecuronium, Venlafaxine, Verapamil, Verteporfin, Vigabatrin, Vinblastine Sulph., Vincristine, Vinorelbine, Vitamin A, Vitamin B, Vitamin B6, Vitamin B12, Vitamin C, Vitamin D, Vitamin E, Voriconazole,

Warfarin

Xylomethazoline Hcl,

Yohimbine,

Zalcitabine, Zidovudine, Zinc Oxide, Zinc Sulph, Ziprazidone Hcl, Zoledronic Acid, Zolmitriptan, Zolpidem, Zopiclone, Zuclopenthixol Dihyd.

The invention will be particularly useful for the delivery of peptide and/or protein drugs. These drugs have very exacting requirements for their formulation in the solid state and could clearly benefit from targeted delivery in the gastrointestinal tract. A most preferred site for their delivery would be the lower GI tract including the colon where the activity of proteases is an order of magnitude lower than in the small intestine, or sites in the distal small intestine where peptide carriers are known to operate. These drugs are also difficult to formulate into tablets because protein molecules may lose their biological activity upon a conformational change that may take place when subjecting the materials to pressure in a tablet press. Formulation in capsules is preferred. Furthermore, protein drugs need special formulation requirements to keep them active in solution and in the stored solid phase. Any excipients added to the formulation to affect drug delivery will potentially be a source of incompatibility or instability. The advantage of this invention for said formulations is in the ability to use proven formulations for the drug with no interaction with the components of the delivery system.

Protein and peptide drugs for which this invention could be useful include insulin, growth hormone (GH), growth hormone releasing hormone (GHRH), calcitonin, epithelial growth factor, vascular endothelial growth and permeability factor (VEGPF), nerve growth factor, cytokines, interleukins, interferons, GMCSF, hormone-like products, neurological factor, neurotropic factor, neurotransmitter, neuromodulator, enzyme, antibody, peptide, proteic fragment, vaccines, immune stimulating or inhibiting factor, heomatopoietic factor, anti-cancer product, anti-inflammatory agent, anti-parasitic compound, anti-microbial agent, cell proliferation inhibitor or activator, cell differentiating factor, blood coagulation factor, immunoglobulin and others hormones and recombinant protein drugs.

The present invention will be also appropriate for delivery of drugs possessing poor bioavailability, specially those whose low bioavailability is caused by extensive first-pass metabolism occurring in the small intestine. Such molecules may be metabolized in intestinal lumen by cytochrome P450 isoenzyme type CYP3A4 or by any other types of cytochrome P450 isoenzymes, existing in a high concentration mainly in the upper GI tract, prior to absorption. Using the delivery system (method and formulation) according to the present invention, dosage form can overtake jejunum and small intestinal, where isoenzyme CYP3A4 can be found in a high concentration, thus releasing the drug in the lower GI tract, where the concentration of CYP3A4 is relatively poor. In this way the effect of the first-pass metabolism (pre-systemic metabolism) of the drug can be decreased and thus drug bioavailability can be improved.

Examples of drugs which are substrates for CYP3A: Alprazolam, Amiodarone, Amitriptyline, Astemizole, Atorvastatin, Budesonide, Bupropion, Buspirone, Caffeine, Carbamazepime, Cerivastatin, Cisapride, Claritromycin, Clomipramin, Clonazepam, Codeine, Cyclosporine, Dexametazone, Dextrometorphan, DHEA, Diazepam, Diltiazem, Disopiramide, Donepezil, Doxycicline, Erytromycin, Estradiol, Ethylestradiol, Felodipine, Fluoxetine, Imipramine, Lanzoprazole, Lidocaine, Loratidine, Lovastatin, Midazolam, Nefazodone, Nicardipine, Nifedipine, Nizoldipine, Norethindrone, Omeprazole, Ondansetron, Orphenadrine, Paroxetine, Progesterone, Pro[afenone, Quethiapine, Quinidine, Rifampin, Sertraline, Sibutramine, Sildenafil, Simvastatin, Tacrolimus, Tamoxifen, Terfenadine, Testosterone, Theophyline, Trazodone, Triazolam, Venlafaxine, Verapamyl, Vinblastine, (R)-Warfarin, Zolpidem.

The invention will be further understood by reference to the accompanying figures, in which:

Figure 1 is a graphical representation of percentage release rate as a function of time for 3 different concentrations of coated Diclofenac tablets with an inner layer containing HPC and no citric acid and an outer layer containing Eudragit E/CaP;

Figure 2 is a graphical representation of percentage release rate as a function of time for 4 different concentrations of coated Diclofenac tablets with an inner layer containing HPC/citric acid/talc and an outer layer containing Eudragit E/CaP;

Figure 3 is a graphical representation of percentage release rate as a function of time for 3 different concentrations of coated Diclofenac tablets with an inner layer containing HPC/citric acid/talc and an outer layer containing Eudragit E/CaP, wherein the ratios of the components of said outer layer are different from that shown in Figure 2.;

Figure 4 is a graphical representation of percentage release rate as a function of time for 4 different concentrations of coated Diclofenac tablets with an inner layer containing HPC/EDTA/talc and an outer layer containing Eudragit E/CaP;

Figure 5 is a graphical representation of percentage release rate as a function of time for 3 different concentrations of coated Diclofenac tablets with an inner layer containing HPC/EDTA 4Na/talc and an outer layer containing Eudragit E/CaP;

Figure 6 is a graphical representation of percentage release rate as a function of time for 4 different concentrations of coated Tramadol tablets with no inner layer and an outer layer containing Eudragit E/CaP;

Figure 7 is a graphical representation of percentage release rate as a function of time for 3 different concentrations of coated Tramadol tablets with an inner layer containing HPC/EDTA and an outer layer containing Eudragit E/CaP;

Figure 8 is a graphical representation of percentage release rate as a function of time for 3 different concentrations of coated Tramadol tablets with an inner layer containing HPMC/EDTA/PEG 400 and an outer layer containing Eudragit E/CaP;

Figure 9 is a graphical representation of percentage release rate as a function of time for 4 different concentrations of coated Tramadol tablets with an inner layer containing HPMC/Citric Acid/talc/PEG 400 and an outer layer containing Eudragit E/CaP;

Figure 10 is a graphical representation of percentage release rate as a function of time for 2 different concentrations of coated Tramadol tablets with different weight of inner layer ratio of HPC/Citric Acid/talc/Aerosil and an outer layer containing Eudragit E/CaP;

Figure 11 is a graphical representation of percentage release rate as a function of time for 2 different concentrations of coated Tramadol tablets with different weight of inner layer ratio of HPC/Citric Acid/talc/Aerosil and an outer layer containing Eudragit E/CaP;

Figure 12 is a graphical representation of percentage release rate as a function of time for 3 different concentrations of coated Tramadol tablets with an inner layer containing HPC/Citric Acid/talc/Aerosil and an outer layer containing Eudragit E/CaP;

Figure 13 is a graphical representation of percentage release rate as a function of time for 4 different concentrations of coated Pyridostigmine capsules with no inner layer and an outer layer containing Eudragit E/CaP;

Figure 14 is a graphical representation of percentage release rate as a function of time for coated Pyridostigmine capsules with an inner layer containing HPC/Citric Acid/talc/Aerosil and an outer layer containing Eudragit E/CaP;

Figure 15 is a graphical representation of percentage release rate as a function of time for 3 different concentrations of coated Pyridostigmine capsules with an inner layer containing HPC/Citric Acid/Aerosil and an outer layer containing Eudragit E/CaP;

Figure 16 is a graphical representation of percentage release rate as a function of time for 3 different concentrations of coated Pyridostigmine capsules with an inner layer containing HPC/Citric Acid/ Aerosil and an outer layer containing Eudragit E/CaP, with a different ratio of the components of the outer layer than that shown in Figure 15.

Figure 17 is a graphical representation of percentage release rate as a function of time for 3 different concentrations of coated Pyridostigmine capsules with an inner layer containing HPC/Citric Acid/Aerosil and an outer layer containing Eudragit E/CaP. As will be noted, the inner layer is thinner than that shown in Figure 16.

Figure 18 is a graphical representation of percentage release rate as a function of time for 3 different concentrations of coated Pyridostigmine capsules with an inner layer containing HPC/Citric Acid/Aerosil and an outer layer containing Eudragit E/CaP . As will be noted the inner layer is thicker than that shown in Figure 16.

Figure 19 is a graphical representation of percentage release rate as a function of time for 3 different concentrations of coated Pyridostigmine capsules with an inner layer containing HPC/Citric Acid/ Aerosil and an outer layer containing Eudragit E/CaP, wherein the components of the outer layer are of a different ratio than that shown in Figures 16-18.

Figure 20 A is a graphical representation of correlation between lag time and the weight ratio of outer layer to citric acid included into the inner layer.

Figure 20 B is a graphical representation of correlation between lag time and the weight ratio of outer layer to inner layer.

Figure 20 C is a graphical representation of correlation between the weight ratio of outer layer to citric acid included into the inner layer and burst time (the time takes to 80% of release).

Figure 20 D is a graphical representation of correlation between the weight ratio of outer layer to inner layer and burst time (the time takes to 80% of release).

Definitions

In the description that follows the following terms are used in order to describe the specification and claims. The following definitions are provided in order to provide clarity to the discussion. Where not specifically indicated all other terms are used in their normal or art-recognized meanings.
The term "delivery device" or "delivery system" is intended to mean a preparation that is designed to deliver a desired agent such as a drug. The preparation can be a simple or complex combination of chemicals, drugs (one or more) or excipients. The delivery can be controlled such that the site or time of drug release is preset by the parameters of the coating layers. Such control can be by chemical or physical means. In this invention "delivery system" and "delivery device" are used interchangeably.
The term "drug" is intended to mean any pharmaceutical or physiological agent, composition, bioactive compound or combination thereof, that is useful in the cure or prevention of any disease, or for any other medical purpose.
The term "particulate" is intended to mean a composition composed of separate particles. In this invention the particles are embedded into a film surrounding the core or capsule. The particles serve as channels for the entry of water into the system. The particles may swell but remain in all cases as separate particles. They do not coalesce into a gel or film in themselves.
In the context of this invention the terms, "coating", "film coating", "membrane", and "layer" are used interchangeably to denote an integral layer of a material that is coated upon another surface. The layer may be formed of one or more materials, several of which may be present as a molecular distribution one in other, while others may be separate, as particles embedded therein.
The term "water-insoluble" means that the material is relatively not susceptible to dissolution in water or aqueous solutions. The term "water-soluble" means that the material is relatively susceptible to dissolution in water or aqueous solutions. The term "hydrophobic" when applied to a coating means that the film is relatively impermeable to the passage of water or aqueous solutions while the term "hydrophilic" when applied to coatings or to particulates means that the material is relatively permeable to the passage of water or aqueous solutions.

The term "embedded" or "embed" means a firm fixation of the material within a coating or layer.

The term "channel" is intended to mean that through which something may flow. The channels in this invention can be filled by the material of the particulates, however, they allow the flow of water and aqueous solutions.

The term "rupturing agent" is intended to mean any pharmaceutically acceptable component added into the inner layer that, by further penetration of water, can attack the outer layer in order to disrupt its integrity and eventually, to cause it to burst.

The term "chelating agent" denotes a component added into the inner layer to compete for a metal ion of an ion cross-linked polysaccharide or a component that modifies cellulose when used as the hydrophilic insoluble particulate in the outer layer.

The term "core" is intended to mean a solid dosage form that contains the active material inside and/or outside, and that may be prepared by a granulation, tabletation, or microencapsulation process. Examples of such a dosage form are; granules, microspheres, microparticles, microcapsules, microbeads, beads, pellets, tablets, and caplets.

The term "capsule" is intended to mean a solid dosage form that is filled by the active material with or without other pharmaceutically acceptable excipients. Examples of "capsule" can be hard gelatin capsules, soft gelatin capsules, starch-based capsules, HPC-based capsules and HPMC-based capsules.

The invention is directed to a delivery system for: 1. the targeted delivery of a pharmaceutical to a particular location in the gastrointestinal tract; 2. time controlled delivery, as the release can be designed to take place after a predetermined time post administration. The delivery system comprises a core containing a pharmaceutical in any desirable formulation that is fitting for said pharmaceutical. The core or capsule, which can be a gelatin capsule, is such that when it would be exposed to aqueous solutions it dissolves and or disintegrates totally within a short period of time. The purpose of this invention is to allow the targeted delivery of agents that are formulated in the core or capsule in a manner in which the drug formulation need not be changed at all. The control of the site of the exposition of the core or capsule to the environment, is afforded, by coatings placed on the outside of the core or capsule, such that the contents of the core or capsule are not affected in any way. In order to achieve the control of time or site of release, the core or capsule is coated with at least two coatings. The outer coating is the layer that controls the time of exposure of the capsule to the aqueous environment by controlling the rate of water entry into the delivery device. Beneath the outer coating the core or capsule is coated with an inner coating that is relatively easily dissolvable in water and contains the agent that will compromise the integrity of the outer coating. Any convenient solid dosage form is useable in this invention including but not limited to granules, microspheres, microparticles, microcapsules, microbeads, beads, pellets, tablets, caplets, hard gelatin capsules, soft gelatin capsules, starch-based capsules and HPC-based capsules and HPMC-based capsules.

The outer coating is one that controls the entry of water into the inner coating and contains, as one of its essential features, a major component that is insoluble in water but that can be readily dissolved upon release of the agent in the inner coat.

In one embodiment of the current invention the outer coating that controls the entry of water, is a film of a water insoluble polymer, embedded with water insoluble but hydrophilic particles. Such a coating is described in US Patent number 5840332 as a coating for tablets or capsules. Said a coating offers many parameters for controlling drug delivery as described in that patent. The identity, weight percent, particle size of the particles, and the identity of the hydrophobic polymer, as well as the coating thickness, are all parameters that control the entry of water through such a coating. We have found that certain embodiments of that coating can be used for this invention. The water insoluble polymer can be any hydrophobic polymer that is insoluble at neutral physiologic pH. Examples of such polymers are ethylcellulose, Eudragit E , Eudragit RL and RS and Eudragit NE. In the case of Eudragit E, the polymer may be the component that is dissolved by the agent in the inner coating since Eudragit E can be rendered soluble by a change in pH to an acidic value. In the case of the other polymers, the polymer serves the function of limiting the entry of water to the system to the channels formed by the particulates while said hydrophilic non water soluble particles will serve the double function of allowing controlled entry of water while also serving as the site for the destructive dissolution of the outer film, allowing the exposure of the core or gelatin capsule to the environment. The particulate compounds may be any of several metal salts of a polysaccharide such as calcium pectinate or calcium alginate.

In one preferred embodiment the outer layer will comprise ethylcellulose embedded with particles of calcium pectinate. The weight percent of the particles may be from 10 to 90, preferably 30-70, while their particle size may range from 10 micrometers to 500 micrometers, more preferably 80-250 micrometers.

In another preferred embodiment, the outer layer will comprise ethylcellulose embedded with particles of calcium alginate. The weight percent of the particles may be from 10 to 90" more preferably 30-70, while their particle size may range from 10 micrometers to 500 micrometers, more preferably 80-250 micrometers.

In another preferred embodiment, the outer layer will comprise Eudragit E embedded with particles of calcium pectinate. The weight percent of the particles may be from 10 to 90, more preferably 30-70, while their particle size may range from 10 micrometers to 500 micrometers, more preferably 80-250 micrometers.

In one preferred embodiment, the outer layer will comprise Eudragit E embedded with particles of micro crystalline cellulose. The weight percent of the particles may be from 10 to 90, more preferably 30-70, while their particle size may range from 10 micrometers to 500 micrometers, more preferably 80-250 micrometers.

The inner coating is a layer that is found beneath the outer layer but above the core or capsule. It is applied to the capsule first. One may optionally apply an undercoat of an inert water soluble film to the core or capsule before applying the inner coating. Materials suitable for such an inert undercoat are hydroxypropylcellulose or similar polymers. The inner layer contains an agent, which when dissolved by water that has entered through the outer coat, will compromise the integrity of the outer coat. The identity of this agent depends on the identity of the components in the outer coat. For example, an agent that changes the pH of the environment outside the capsule is suitable for the dissolution of films that are based of Eudragit E or other acid soluble films. The embodiments of the outer coat that contain Eudragit E, whether they contain calcium pectinate, alginate or micro crystalline cellulose as the particulate that forms the channels for water entry, can use an inner film of a water soluble polymer such as hydroxypropylcellulose (HPC), polyvinylpovidone (PVP), hydroxypropylmethylcellulose (HPMC), hydroxyethylcellulose (HEC), and carboxymethylcellulose (CMC) which contains citric acid, tartaric acid, or other suitable non volatile organic acids in a solid solution. The weight percent of organic acid dispersed in the inner film can range from 10% to 90%, while the weight percent of water soluble polymer can range from 2% to 40%. In one preferred embodiment of the inner coat one has 75% of citric acid dissolved or dispersed in an inner coating layer of 32 mg/cm$^2$ containing 4% of hydroxypropylcellulose.

An alternate embodiment of the inner coating, is one in which the inner coating contains an agent that reacts with a major component of the outer membrane rendering it readily soluble in water. Examples of such agents are sequestering agents or chelating agents that can remove the metal from an insoluble metal salt, rendering the anionic part of that salt soluble. In a preferred embodiment, the sequestering agent may be ethylenediaminetetraacetic acid (EDTA) salts such as the tetra sodium salt, the disodium salt or the disodium calcium salt or salts of oxalic acid. These agents react with, for example, the calcium in calcium pectinate particles, leaving behind small particles of pectin or with the calcium in calcium alginate particle leaving behind alginic acid. Both pectin and alginic acid are readily soluble at the physiological pH's of 6.5 - 8. Since the outer film comprises a very large percentage of the particulate matter (10 % to 90 %), the total dissolution of the calcium pectinate as pectin totally compromises the integrity of the film, which in essence disintegrates. The inner film has already dissolved, thereby leaving the core or gelatin capsule exposed to the aqueous environment. The entire drug load is thus released readily.

In a preferred embodiment, the inner coating layer contains from 10% to 90% of disodium EDTA. A most preferred embodiment contains about 80% disodium EDTA in an inner coating layer of about 20 hydroxypropylcellulose of about mg/cm$^2$.

An optional enteric coating overlaying the outer membrane may be applied. The enteric coat will protect against the adverse effects of the acid pH in the stomach. For certain embodiments the enteric coat may be necessary to prevent premature drug release caused by interactions of components of the outer coat with the acid of the stomach. Any enteric coating material known in the art may be used for the enteric coat. Eudragit L is the most preferred enteric coating.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

EXAMPLES

1. Delivery system according to the present invention performed on a non-disintegrating core

Materials

The following materials were used for preparing the different formulations;
Calcium pectinate powder containing 4% calcium (CaP, Lot-Nr.-2091889, food grade Genu-Copenhagen Pectin- Denmark);
Micro-crystalline cellulose (Emcocel 90M, Lot-Nr.-9s6073, BP grade, Mendel-Finland);
Micro crystalline cellulose (Avicel PH 102, Lot-Nr.-7806C, NF grade, FMC);
Ethylcellulose (EC-N7 NF, Lot-Nr.-K110013T02, USP grade, Dow-USA);
Lactose (Lot-Nr.-829333, BP grade, Borculo Whey Products);

Starch (Lot-Nr-604022, NF grade, Colorcon-USA);
Polyvinylpyrolidone (PVP 90F, Lot-Nr.-80-6936, USP grade, BASF-Germany);
Cross polyvinylpyrolidone (CPVP, Lot-Nr.-130766, NF grade, BASF-Germany);
Mg stearate (Lot-Nr.-E#672, USP grade,);
Eudragit E 100 (Eud.E, Lot-Nr.-8360801021, Rohm Pharma- Germany);
Hydroxypropyl methyl cellulose (HPMC, Methocel E5, 2910/5, Lot-Nr.-LNJ408, USP grade, Dow-USA);
Hydroxypropyl cellulose (HPC EF, Lot-Nr.-6237, NF grade, Aqualon,-Netherlands);
Ethylenediamine tetraacetic acid disodium (2Na EDTA, Lot-Nr.-390734/1, Fluka);
Citric acid (Lot-Nr.-K9107234, USP grade, Merck-Germany);
Cilicone dioxide (Aerosil, Lot-Nr. R24551326, NF grade, Merck-Germany); and
Talc (Lot-Nr.- K2431170, USP grade, Merck-Germany).

The following materials were used as the model active materials:

Sodium diclofenac (Lot-Nr.-24611, BP grade, Secifarma-Italia); and
Tramadol hydrochloride (HCl) (Lot-Nr.-J136, USP grade, Protocchemic-Switzerland).

Ethyl alcohol was USP grade.

Methods

In this series of experiments, sodium diclofenac and tramadol were used as a representative model for active material. The formulations and the characters of the tablets containing either sodium diclofenac or tramadol are summarized in Table 1.

*Preparation of sodium diclofenac-containing core*

Wet granulation process was used to prepare the cores. The granulation was performed manually for both sodium-diclofenac as well as CaP using a mortar and pestle. The granulation of sodium-diclofenac was carried out as follows: 0.9g of ethylcellulose (EC 7) was dissolved in 15 ml of ethanol. 45 g of sodium-diclofenac and 2.25 g of CPVP were placed in the mortar and the solution of the EC 7 was added slowly. The mixture was well mixed and dried at 40°C for 16 hours.

The granulation of the starch/lactose was carried out as follows: 1 g of PVP K90F was dissolved in 10.0 g of water. 70 g of lactose was mixed with 30.0 g of starch. The PVP solution was added slowly. The mixture was well mixed and dried at 95°C for 16 hours.

The granules (of both active material as well as starch/lactose) and other components (table 1) were transferred to a polyethylene bag and mixed for 20 minutes. 0.6g of magnesium stearate was added and the blend mixed for another 2-3 minutes. Biconvex cores of 7 mm diameter were compressed automatically using a Wick Ges.mbh single punch tablet press. The weights of cores ranged between 205 to 209 mg. The hardness of the cores was tested using a Vanderkamp VK200 Hardness Tester and it ranged from between 9.3 to 12.0 KP.

*Preparation of tramadol HCl-containing cores*

Wet granulation process was used for preparation of the tramadol granules. Ethanolic solution of ethylcellulose (2 g EC7/25.0 ml ethanol) was slowly added to the tramadol (100 g) and CPVP (5.0 g), and the granulation was performed manually using a mortar and pestle. The resultant granules were dried in an oven at 35°C for about 16 hours.

Tramadol HCl containing tablets were prepared by dry mixing of tramadol granules with other components (table 1) except Mg stearate, in a plastic polyethylene bag for 20 minutes. Mg stearate (0.6 g) was added to the blend and the mixing was continued for additional 2 minutes.

Biconvex cores with a diameter of 6 mm were pressed automatically in a Wick Ges.mbh single punch tablet press. The average weight of the cores was 101.6 mg. The hardness of the cores was tested using a Vanderkamp VK200 Tester and the average was calculated to be 5.8 KP. Table1: Formulation used for Na diclofenac and tramadol HCl containing cores

| Raw material | Na-diclofenac - % | | Tramadol - % | |
|---|---|---|---|---|
| | % in granulate | % in formulation | % in granulate | % in formulation |
| Granulate | Form. 412- 27,42 | | Form.438- 61412-111 | |
| A M | 92.3 | 10.2 | 93.5 | 46.8 |
| CPVP | 5.8 | 0.6 | 4.6 | 2.3 |
| EC 7 | 1.8 | 0.2 | 1.9 | 0.9 |
| Granulate lactos (412-20,31,34) | | 68 | | |
| Lactos | 69 | 46.9 | | |
| Starch | 30 | 20.4 | | |
| PVP K90 F | 1 | 0.7 | | |
| Active Material (AM) | 11.0 | | 50.0 | |
| Avicel PH102 | ----- | | 34.5 | |
| Emcocel 90M | 15 | | ----- | |
| CPVP | ------ | | 10 | |
| PVP | 5 | | 5 | |
| Mg Stearate | 1 | | 0.5 | |
| Tablets Form. | 412-41,45 | | 412-95,114 | |
| Hardness,kp | 9.3-12 | | 5.8-6.5 | |
| Diameter,mm | 7 | | 6 | |
| Total Weight,mg | 205-209 | | 101-105 | |

*General coating process:*

The coating suspension was stirred vigorously throughout the coating process to prevent the deposition of particles. The coating process was performed on 100g core tablets. The coating system consisted of a perforated pan coater, a peristaltic pump (Masterflex, Digital Console Drive, Cole-Palmer Instrument Company) and the spraying nozzle. The spraying nozzle was composed of a "Y" connector PE tube fixed on one end to the air supplying system and the other end to the coating suspension through the peristaltic pump and a stainless steel tip of 1.2 mm fixed at the head of the "Y" connector tube. The spraying nozzle was adjusted to aim at the falling cores in the upper part of the pan and a fine jet was sprayed on continuously at a pressure of 0.4-0.5 bar. The spray rate was adjusted to 3 ml/min. The coating pan was rotated at 18 rpm to provide continuous flow of the tablets. The air flow rate through the coater chamber was regulated in the range of 2.75-2.85 m/s which was kept constant throughout the coating process. At the end of the process the coated tablets were discharged and spread out on a sieve and finally dried for 16 hours at temperature 35°C in a drying oven.

*Inner film coat containing hydroxylpropyl cellulose (HPC) on Na-Diclofenac cores:*

Coating suspension: HPC (Klucel EF) (table 2) was dissolved in Ethanol (100 ml) while continuous stirring occurred at 500 rpm.
Citric acid-containing coating: 12.0g of Citric acid was added and after complete dissolution of citric acid, 4.0g talc was added with continuous stirring at 500 rpm.
Ethylenediamine tetraacetic acid disodium (EDTA-2Na)-containing coating: 48.0g EDTA-2Na was milled to particle size <150u and dispersed in Klucel EF solution with continuous stirring at 500 rpm.
The coating process was carried out using a perforated pan coater where a Drum "D", Coater and a Dize-Schlick model 930/3, 0.8mm 20939 were assembled. The coating was performed at 22-25°C. Speed of rotation (21-32 rpm) was changed according to the change of flowability and rolling of tablets.
The spray rate of the suspension was 3.5-4.5 ml/min, according the wetness and adhesiveness of the tablets. The spraying was stopped when the tablets were stuck together and renewed after separation. The air pressure was 0.8 bar.

*Inner film coat containing HPC on Tramadol HCl cores:*

Coating suspension: 4.0g HPC (Klucel EF) dissolved in 100ml Ethanol while continuously stirring at 700 rpm. Citric acid-containing coating: 12.0g of Citric acid was added and after complete dissolution of citric acid, 2.0g Talc and 2.0g Aerosil were dispersed while continuously stirring at 700rpm. For a thicker coating, a double amount of suspension was used.
EDTA-2Na-containing coating: 48.0g EDTA $Na_2$ was milled to particle size <150u and dispersed in HPC EF solution with continuous stirring at 700 rpm.
General coating process of the inner layer containing HPC: Coating was performed at temperature 21-23°C and the pan rotation was 40 rpm.

*Inner film coat containing hydroxypropyl methyl cellulose (HPMC) on Tramadol HCl cores:*

Citric acid-containing coating: 10.0g Methocel E5 and 1.0g PEG-400 were dissolved in a mixture of 38g distilled water and 131g Ethanol, 10.0g Citric acid were dissolved in the Methocel solution, 5.0g talc was added with continuous stirring at 700 rpm.
EDTA $Na_2$ coating: 10.5g Methocel E5 and 1.1g PEG-400 were dissolved in a mixture of 160g distilled water and 40g ethanol, 42.0g EDTA $Na_2$ (particle<150u) was dispersed in the Methocel solution with continuous stirring at 700 rpm.
General Coating process of the inner layer containing HPMC: the coating process was performed at temperature 35-45.°C and the pan rotation was 35-40 rpm.

*Coating process of the outer film coat:*

Preparation of the coating suspension: CaP powder underwent fractionation using a sieve shaker (Ari J. Levy, Laboratory Equipment LTD) and a sieve of 149μ (ASTM 100, 8" diameter) in order to obtain only the fractions of <149μm particle size. The coating suspension was prepared by dissolving 27.0g Eudragit E in 163g ethanol and 63.0g (ratio3: 7) or 27.0g (ratio1:1) of fractionated CaP was added while stirring (700 rpm.) to the solution.
Coating process: The spray rate was adjusted to 3 ml/min and the tablet temperature was kept at 24°C-26°C.

*Drug release assessments*

Dissolution studies were performed in intestinal fluid TS (phosphate buffer pH 7.5 without enzymes) using a Vankel 7000 dissolution tester. One tablet was placed in 900 ml intestinal fluid TS and stirred by paddle at 50 RPM. The solutions were kept at 37°C by a Vankel VK650A heater/circulator. Samples of 3 ml were taken using a Vankel VK8000 Autosampler, at intervals of 30-60 minutes up to suspected 80% active material release time, followed by intervals of 2 hours up to suspected 100% release time. The actual determinations of the release of the drugs (dissolution results) from the tablets were carried out using a HP 8452A Diode-Array Spectrophotometer. The drugs released were quantified using a calibration curve obtained from the standard solution, in intestinal solution TS, in the concentration range of 0-80 ppm for tramadol HCl and 0-50 ppm for Na-diclofenac.

Results and discussion

The results of the dissolution test of both coated and uncoated sodium diclofenac tablets are shown in the Table 2.

Table 2: Coated tablets containing Diclofenac Sodium: composition and release profiles.

| # Form | Inner Coat | | | Outer coat | | Lag time (hours) | %release after 1h | %release after 2h | Time of release 80% (hours) |
|---|---|---|---|---|---|---|---|---|---|
| | Coating type and ratio (%) | Coating weight | | Coating type and ratio (%) | Coating weight (mg) | | | | |
| | | mg | mg/cm² | | | | | | |
| I | HPC EF— 50 | | | Eudragit E—30 | 14 | 2 | 8 | 19 | 9 |
| | Talc ——— 50 | 4 | 2 | CaP——— 70 | 26 | 3 | 3-4 | 8 | >10 |
| | | | | | 42 | 4 | 1-2 | 5-8 | >10 |
| II | HPC EF— 20 | | | Eudragit E—30 | 28 | - | 64-75 | 90-92 | 1.5 |
| | Citric acid— 60 | 16 | 9 | CaP——— 70 | 55 | 2 | 59-78 | 63-89 | 1 |
| | Talc ——— 20 | | | | 73 | 2.5 | 48-77 | 87-89 | 1 |
| | | | | | 87 | 3 | 56-16 | 76-36 | 5, >5 |
| III | HPC EF— 20 | | | Eudragit E—50 | 11 | - | 19-89 | 95-99 | 0.5-1 |
| | Citric acid— 60 | 14 | 8 | CaP——— 50 | 24 | - | 3-4 | 5-6 | 10,>10 |
| | Talc ——— 20 | | | | 31 | -- | 3 | 3-4 | >12 |
| IV | HPC EF— 12.9 | | | Eudragit E—30 | 17 | 0.5-1 | 8-17 | 25-70 | 3-11 |
| | EDTA-2Na-80.6 | 20 | 11 | CaP——— 70 | 30 | 1.5 | 10-67 | 23-82 | 1.5-10 |
| | Talc ———20 | | | | 53 | 3 | 15-57 | 43-70 | 3.5, >9 |
| V | HPC EF—13.3 | | | Eudragit E—30 | 19 | 1-1.5 | 9-13 | 25-28 | 7 |
| | EDTA-4Na-50.0 | 7 | 4 | CaP——— 70 | 34 | 2.5 | 5-7 | 17-9 | 9-10 |
| | Talc ———16.7 | | | | 49 | 4.5 | 6 | 13 | >10 |

EP 1 731 142 A1

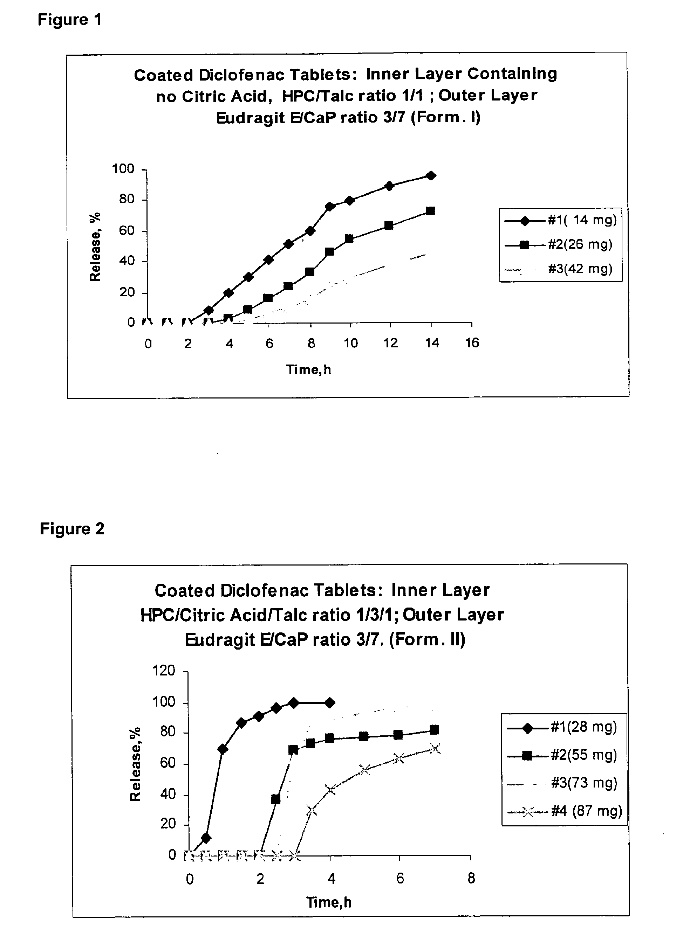

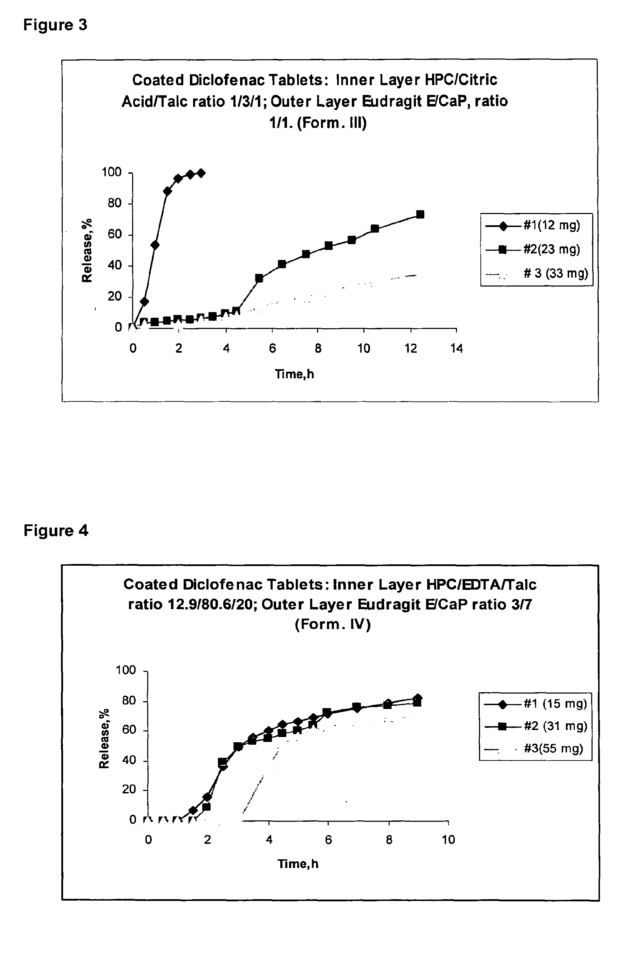

As it is shown, a lag time of 2.5 and 3 hours can be obtained upon using a thickness of 70 and 90 mg of the outer coating film respectively (see formulation II in Table 2 and Figure 2). The mechanism of the release is based on the fact that citric acid is dissolved, upon the penetration of the water into the inner film coat, to form an acidic pH environment under the outer film coat in which Eudragit E in the outer film coat can be subsequently dissolved. Conversely, the tablets containing no citric acid in the inner layer (see formulation I in Table 2 and Figure 1) resulted in a typical sustained release continuing for over ten hours. This fact indicates that the outer film keeps its integrity where no organic acid exists in the inner layer, thus a typical sustained release could be resulted. Table 2 also shows the results of the release of diclofenac where the inner layer contains EDTA, either disodium or tetra-sodium salt. As it is shown this access may also result in a delayed burst release even though the burst is less sharp, as compared to the tablets coated with citric acid-containing inner coat. This fact implies that, again, a disintegrity of the outer coat can take place owing to the reaction occurring between EDTA of the inner layer and CaP existing in the outer coat. This fact results in dissolution of CaP from the outer coat and thus eventually pores that are subsequently formed enable the fast release of the active material. Figures 1-5 show the release profiles of formulations I-V.

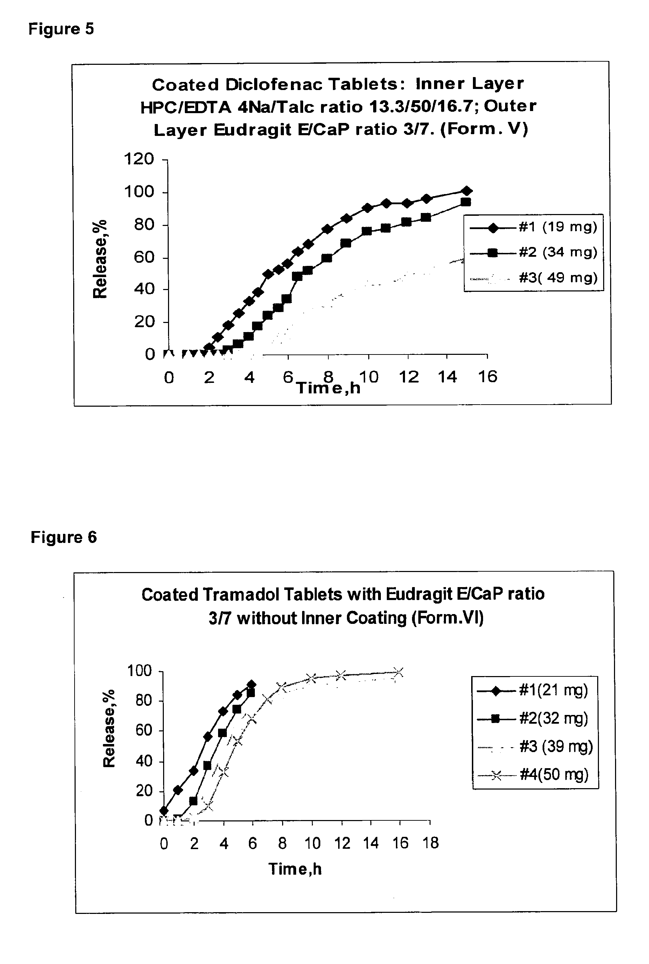

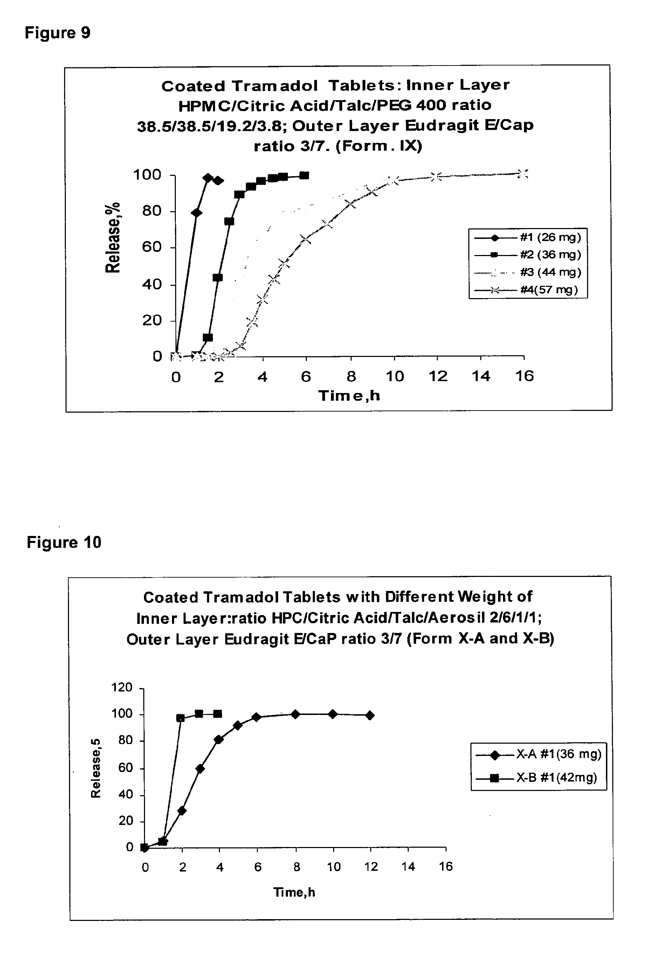

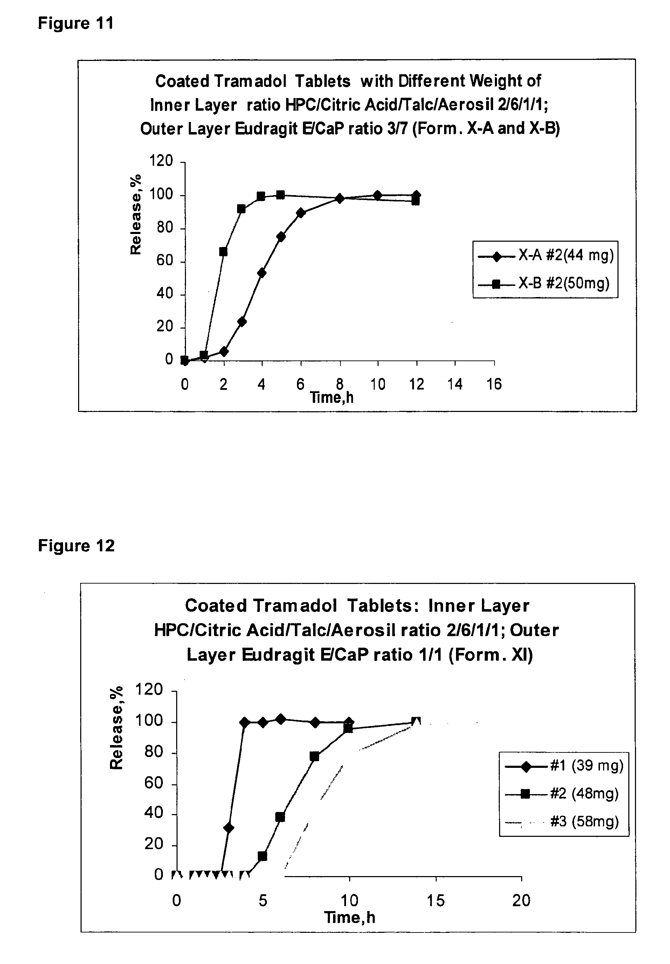

Table 3 summarizes the results of both lag time as well as the release profile of tramadol from disintegrating-immediate release core coated with either outer coat alone or both inner and outer coat. The results show again that the inner coat containing citric acid is involved in the destruction of the outer coat and consequently results in a burst release. The tablets without the inner layer demonstrated again a typical sustained release where the lag time was affected by the weight of the outer coat. The release profile of tramadol from coated tablets without inner layer, is shown in figure 6. Figures 10 and 11, show the release profile of coated tablets with different weights of inner coating layer (Formulations X-A and X-B) accompanied with low (Figure10) and high weight (Figure 11) of outer coat.

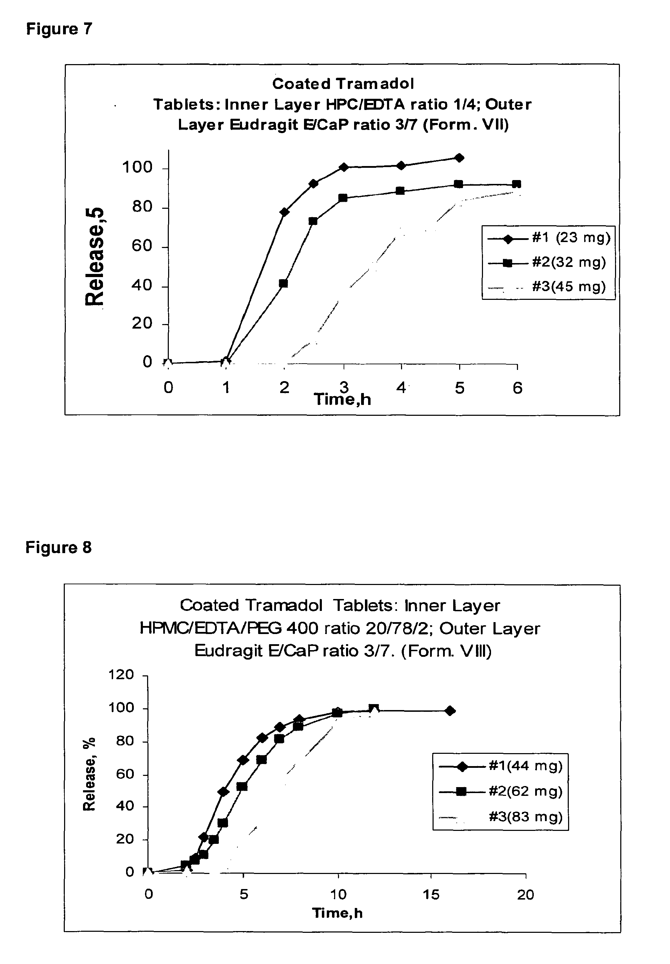

The effect of 2Na-EDTA on the burst release, appeared to be dominant as compared to the tablets containing no inner layer, although it was found to be less significant than citric acid. The effect of 2Na-EDTA is produced by the lowering of the pH and consequently causing the destruction of the outer film coat. Figure 7 shows the effect of the presence of 2Na-EDTA in the inner coat on the release profile of tramadol for different weights of the outer coat.

The effect of the thickness (the film coat weight) of the inner film coat, and thus the amount of the citric acid has also been assessed. For this purpose the tramadol containing cores were coated with increasing weights of the inner layer while maintaining a constant weight ratio of HPC to citric acid. The cores with inner layer weights of the 14 mg and 28 mg were tested. The coated cores were then coated with the outer coating film. The results show that the thicker the inner film coat the faster the release of tramadol, indicating faster and/or better destruction of the outer film coat. The release profile of these tablets is shown in Figure 10 (X-A and X-B) for different weights of the inner coat.

The effect of the weight fraction of CaP particles imbedded into the outer film coat is shown in Figures 10 (X-B) and 12 for the weight ratios of Eud.E/CaP of 3/7 and 1/1 respectively, where a higher weight of the inner coat (26 mg) was used. The data is summarized in Table 3.

Table 3: Coated tablets containing Tramadol: composition and release profiles.

| # Form. | Inner Coat | | | Outer Coat | | Lag time (h) | Release after 1h (%) | % Release after 2h | Time of release 80%(h) |
|---|---|---|---|---|---|---|---|---|---|
| | Coating type and ratio(%) | Coating weight | | Coating type and ratio(%) | Coating weight (mg) | | | | |
| | | mg | mg/cm² | | | | | | |
| VI | | | | Eud.E-30 CaP--70 | 21 | 0.5 | 20 | 47 | 4 |
| | | | | | 32 | 1 | 10-16 | 35-38 | 4.5 |
| | | | | | 39 | 1.5 | 12-17 | 46-47 | 5.5 |
| | | | | | 50 | 2 | 10-11 | 30-34 | 5 |
| VII | HPC_EF-----20 EDTA_2Na-80 | 25 | 19 | Eud.E-30 CaP--70 | 23 | 0.5-1 | 5-82 | 76-92 | 2 |
| | | | | | 32 | 1 | 16-55 | 80-92 | 2 |
| | | | | | 45 | 2 | 10-62 | 58-82 | 2-4 |
| VIII | HPMC-------20 EDTA_2Na-78 PEG400----2 | 20 | 16 | Eud.E-30 CaP--70 | 44 | 2 | 17-26 | 41-58 | 4-5 |
| | | | | | 62 | 2 | 10 | 30 | 5 |
| | | | | | 83 | 2.5 | 1-3 | 5-6 | 8 |
| IX | HPMC-----38.5 CitricAcid-38.5 Talc-------19.2 PEG400--3.8 | 26 | 20 | Eud.E-30 CaP---70 | 26 | --- | 58-100 | 94-100 | 1-1.5 |
| | | | | | 36 | 1 | 28-58 | 88 | 2 |
| | | | | | 44 | 1.5 | 14-36 | 46-66 | 3.5-5.5 |
| | | | | | 57 | 2 | 13-24 | 30-55 | 6 |
| X-A | HPC_EF-----20 CitricAcid----60 Talc---------10 Aerosil-------10 | 14 | 11 | Eud.E-30 CaP---70 | 36 | 0.5 | 12-20 | 36-56 | 4-5 |
| | | | | | 44 | 1-2 | 4-7 | 5-26 | 4-5 |
| X-B | | 28 | 21 | Eud.E-30 CaP---70 | 42 | 0.5 | 50-51 | 96-98 | 1.5 |
| | | | | | 50 | 1 | 30-39 | 70-87 | 2-3 |
| XI | | 26 | 20 | EudrE-- 50CaP----50 | 39 | 2.5-3 | 82-98 | 100 | 1 |
| | | | | | 48 | 4-5 | 20-39 | 20-71 | 3 |
| | | | | | 58 | 7 | N.A. | 43-45 | 4 |

It will be noted that the weight ratio of Eud.E/CaP is important since it can effectively control the lag time. This fact is based on our previous findings (patent TCDS) showing that the lower the weight fraction of the particles in the film coat, the longer the lag time and the slower the release of the active material. As one can see (Table 3) the outer coating with the weight ratio of Eud.E/CaP of 1/1 resulted in a longer lag time as compared to the weight ratio of 3/7, where the same weights of both inner and outer coat were used. Despite the effect on the lag time no effect of the weight ratio of the CaP particles on the release profile was seen.

In another study, hydroxypropyl methyl cellulose (HPMC) was used as a binder in the inner layer formulation containing either citric acid or 2Na-EDTA. The results are demonstrated in Figures 8 and 9 for citric acid and EDTA respectively. As one can see, no significant difference between HPC (Figures 11, 7) and HPMC (Figures 8 and 9) can be found in both lag time and release profile. This finding relates to the high solubility of HPMC used for this purpose which is as high as that of HPC.

Conclusion

The examples show that the drug delivery system according to the present invention can be used as a timed controlled delivery system, which can be directed to the targeted delivery of an active material to a particular location in the gastrointestinal tract. The delivery system comprises either a non-disintegrating or disintegrating core containing the active material with optionally other excipients. The core is first coated with an inner layer which comprises at least on rupturing agent and a water soluble binder. The core is further coated with an outer layer, comprising hydrophilic water insoluble particulates embedded in a relatively hydrophobic substantially water insoluble polymeric matrix. Accordingly, the outer layer is responsible for controlling the entry of water into the inner coat and can thus adjust the lag time. Furthermore, through the penetration of water through the outer layer, the inner layer is dissolved and thus can chemically disrupt the integrity of the outer layer. The agents which can be used for this purpose are either a pH lowering agent, dissolving the polymeric matrix of the outer layer, or a chelating agent that reacts with water insoluble particulates in the outer layer rendering them readily soluble in water. The failure of the outer layer then affords total delivery of the drug load at the predetermined site or time.

Accordingly, the nature of the rupturing agent in the inner coating will be determined by the nature of the outer coating. Delay of drug release from dosage form (lag time) can be controlled by varying the parameters of the outer film coat, such as the thickness and the weight ratio of the particulates. In addition, the lag time can be controlled by the thickness of the inner coating layer, and thus the amount of the rupturing agent in it, as well.

In this manner, the drug is therefore delivered to the desired site without any need to be reformulated with excipients of the delivery system. Furthermore, the drug load and its formulation can be kept totally separate from the components of the delivery system, for example where a gelatin capsule is used.

2.: Delivery system according to the present invention performed on a hard gelatin capsule

In the following series of examples, pyridostigmine was used as a model for the active material. The following study and experiments have been performed on hard gelatin capsules, filled with pyridostigmine granulated with other excipients. The capsule may include additional excipients imparting to the drug any desired improved properties such as stability or absorption enhancement. Other excipients which may be included inside the capsule can be a flow regulation agent, filler, lubricant, disintegrant, solubilizer, suspending agents, dispersing agents, surfactant, and others.

MATERIALS

*Granulate Formulation:*

Active ingredient:

Pyridostigmine Bromide (Lot-Nr 98105/23,USP grade, Orga.Synthetic Industries)
Excipients:

* Filler: Calcium Hydrogen phosphate. Dihydrate (Lot-Nr 92500 , BP grade, Riedel de Haen)
* Binder: Sorbitol (Lot-Nr 2870870 Sigma)
* Disintegrante: Crosspovidone (CPVP, Lot- Nr 130766, USP grade, BASF Germany)

Inner layer

* Polymer: Hydroxypropyl cellulose (HPC EF, Lot-Nr. 6237,NF grade, Aqualon Netherlands)
* PH-lowering agent: Citric acid anhydrous (Lot-Nr. K91072347, USP grade, Merk-Germany)
* Glidants: - Silicone dioxide (Aerosil, Lot-Nr. R24551326, NF grade, Merk-Germany)
Talc (Lot-Nr. K2431170, USP grade, Merk Germany)
* Ethyl alcohol was USP grade

Outer film coat:

* Eudragit E100 (EudE, Lot-Nr. 8360801021,Rohm Pharma-Germany)
* Calcium Pectinate powder containing 4% Calcium (CaP, Lot-Nr. 2091889, food grade Genu-Copenhagen Pectin-Denmark)

METHODS

*Preparation of pyridostigmine granulate*

Wet granulation process was used to prepare the pyridostigmine granules.
The granulation solution of Pyridostigmine was carried out as follows:

4g (2%) Pyridostigmine was dissolved in 16g purified water. 152g (76%) Calcium Hydrogen phospho. Dehydrate, 14g (7%) Crosspovidone and 30g (15%) Sorbitol were mixed for 5mn in a plastic polyethylene bag. This mixture was transferred to a mortar and pestle and the solution of Pyridostigmine was added slowly. This wet granulate was well mixed and oven dried at 65°C for 16 hours. After oven drying, the resulting dry granulate was milled manually using a mortar and pestle. The milled dry granulate was sieved through a 420μ sieve.

*Filling active Capsule*

The capsules were filled with dry milled granulate manually by using filling system Hanin Technical Supply LTD Capsule loader 100 capsules size 4 (CH100-4) Feton International. S.A.

*Coating process*

*Coating system*

The coating suspension was kept stirred vigorously using a magnetic stirrer (Heidolph MR 3001) throughout the coating process to prevent the deposition of particles. The coating system consisted of a perforated pan coater, a peristaltic pump Masterflex, Digital Console Drive, Cole-Palmer Instrument Company) and the spraying nozzle. The perforated pan coater was a Drum "E" coater. The spraying nozzle used for coating the inner layer was a Dize-Schlick model 930/3, 0.8mm 20939. The spraying nozzle used for coating outer layer was composed of a "Y" connector PE tube fixed on one end to the air supplying system and the other to the coating suspension through the peristaltic pump and a stainless steel tip of 1.2mm fixed at the head of the "Y" connector tube. The spraying nozzle was aimed at the falling capsules in the upper part of the pan and a fine jet of coating suspension was sprayed on continuously.

*Preparation of inner layer coating*

In this study the inner coating layer was composed of citric acid as the pH lowering agent, and low molecular weight Hydroxypropyl cellulose EF (HPC) as water soluble binder. After water penetration into the inner film coat citric acid forms an acidic pH environment under the outer film coat, leading to dissolution of the pH dependant polymer of outer film (Eudragit E100) and thus burst of the coating.

*Preparation of the inner layer suspension*

*Inner layer without binder:*

24g Citric acid anhydrous was dissolved in 150 g Ethanol using a magnetic stirrer (500-rpm). After complete dissolution 4g Talc and 4g Aerosil were added with continuous stirring at 500rpm.

*Inner layer containing binder (HPC.EF):*

1.4g HPC.EF was dissolved in 120g Ethanol and 30g water, and 26.2g Citric acid anhydrous was dissolved in HPC.EF solution using a magnetic stirrer (500-rpm). After complete dissolution 3.7g Talc and 3.7g Aerosil were added with continuous stirring at 500rpm.

*Inner layer coat containing binder (HPC. EF) but not Talc:*

2.8g HPC.EF was dissolved in 240g Ethanol and 60g water, and 52.4g Citric acid anhydrous was dissolved in HPC.EF solution using a magnetic stirrer (500-rpm). After complete dissolution 14.8g Aerosil were added with continuous stirring at 500rpm

*Coating conditions-inner layer.*

Perforated Coater Drum: "E"
Inlet air (° C): 32-38
Outlet air (° C): 24-30
Product temperature (° C): 30
Pan rotation speed (rpm): 20
Suspension flow rate (ml/min): 2.5-3
Spray air pressure (bar): 0.5
At the end of the process the coated capsules were discharged and spread out on a sieve and finally dried 16 hours at temperature 35°C in a drying oven.

*Outer layer*

The outer layer film was formed from a relatively hydrophobic polymer with pH dependent solubility (Eudragit E100, Eud.E) in which was embedded non-soluble but hydrophilic particles (Calcium Pectinate, CaP). The outer coating layer delays the drug release by controlling of water penetration into the inner acid-including layer. The lag time of coating burst and subsequently drug release was adjusted by controlling both the thickness of the outer film coat as well as the weight ratio of the polymer and CaP particles in the film coat. The rate of active material release depends also on the weight ratio between the outer film coat and the citric acid.

Preparation of outer film coat

*Coating suspension preparation*

Calcium Pectinate powder underwent fractionation using a sieve shaker (Ari J. Levy, Laboratory Equipment LTD) and a sieve of 149$\mu$ (ASTM 100, "8" diameter) in order to obtain only the fractions of <149$\mu$ particle size. The coating suspension was prepared by dissolving Eudragit E 100 in ethanol and dispersing Calcium Pectinate in the obtained solution while stirring (700rpm). Coating suspensions with different weight ratios of Eudragit E to CaP were checked in these studies:

Ratio 3:7: 27g Eudragit E was dissolved in 163g ethanol and 63g fractionated CaP was added.
Ratio 7:3: 56g Eudragit E was dissolved in 340g ethanol and 24g fractionated
CaP was added.
Ratio 1:1: 56g Eudragit E was dissolved in 340g ethanol and 56g fractionated CaP was added.
Ratio 1:1: 39.6g Eudragit E was dissolved in 240g ethanol and 39.6g fractionated CaP was added.

The coating suspension was kept stirred vigorously using a magnetic stirrer (Heidolph MR 3001) throughout the coating for homogenous particles distribution in the suspension.

*Coating conditions-outer layer:*

Perforated Coater Drum: "D"
Inlet air (° C): 26-40
Outlet air (° C): 22-30
Product temperature (° C): 25-33

Pan rotation (rpm): 20
Suspension flow rate (ml/min): 3.3-4
Spray air pressure (bar): 0.4
At the end of the process the coated capsules were discharged and spread out on a sieve and finally dried 16 hours at temperature 35°C in a drying oven

*Dissolution Test*

Dissolution studies were performed in intestinal fluid TS (phosphate Buffer pH 7.5 without enzymes) using a Vankel 7000 dissolution tester. One capsule was placed into a spring in 500ml intestinal fluid TS and stirred by paddle at 50 RPM. The solutions were kept at 37°C by a Vankel VK650A heater/circulator. Samples of 3 ml were taken using a Vankel VK8000 Autosampler , at intervals of 30-60min up to suspected 80% active material release time. The actual determinations of the release of the drugs (dissolution results) from the capsules were carried out using a HP 8452A Diode-Array Spectrophotometer. The drugs released were quantified using a calibration curve obtained from the standard solution, in intestinal solution TS , in the concentration range of 0-50 ppm for Pyridostigmine bromide.

*Citric acid Content Analysis*

The amount of citric acid per capsule was checked in the various batches, according to the BP 1998 assay (p. 350) which was modified to suit the required analysis. In short, 10 precoated capsules were dissolved in 100ml water. The solution was titrated with 0.1M NaOH VS solution, using phenolphthalein as indicator. A solution of 10 uncoated capsules was titrated in the same manner. The amount of citric acid per capsule was calculated according to the BP assay:

$$\text{Citric acid amount per capsule (cf. Table 1)} =$$

$$(\text{NaOH volume }_{coated\ solution} - \text{NaOH volume }_{uncoated\ solution})*6.403/10$$

$$= \frac{\text{precoated solution NaOH volume} - \text{uncoated solution NaOH volume}}{10} \times 6.403$$

RESULTS AND DISCUSSION

The release profile of Pyridostigmine from coated capsules without inner film coat and with inner film coat, containing citric acid, are respectively shown in Table 4.

Table 4: Coated capsules containing Pyridostigminel: composition and release profiles.

| # Form. | Inner Coat — Coating type and ratio(%) | Inner Coat — Coating weight (mg) | Inner Coat — Coating weight (mg/cm²) | Outer Coat — Coating type and ratio(%) | Outer Coat — Coating weight (mg) | Lag time (h) | Release after 1h (%) | % Release after 2h | Time of release 80%(h) |
|---|---|---|---|---|---|---|---|---|---|
| XII | | | | Eud.E-30 CaP—70 | 30 | 1.5-1 | 35-30 | 67 | 3.5-3 |
| | | | | | 34 | 2 | 36-30 | 52 | 5-3.5 |
| | | | | | 40 | 3-2 | 40-75 | 70-100 | 1.5-2.5 |
| | | | | | 44 | 3 | 40 | 68-88 | 2-2.5 |
| XIII | HPC——4 Citric Acid-75 Aerosil——10.5 Talc——10.5 | 36 | 16 | Eud.E-50 CaP—50 | 75 | 6-7 | 45-57 | 75-95 | 1.5-2.5 |
| XIV | HPC——4 Citric Acid-75 Aerosil——21 | 60 | 27 | Eud.E-30 CaP—70 | 36 | 2-1.5 | 38-7 | 86-27 | 6-1.5 |
| | | | | | 69 | 6-5 | 99-24 | 99-84 | 2-1 |
| | | | | | 84 | 14-7 | 5-4 | 98-50 | 4-2 |
| XV | HPC——4 Citric Acid-75 Aerosil——21 | 60 | 27 | Eud.E-50 CaP—50 | 42 | 1-1.5 | 85-100 | 100 | 0.5 |
| | | | | | 90 | 4.5-4 | 96-72 | 100 | 1.5-0.5 |
| | | | | | 110 | 8 | * | 20-31 | 4-5 |
| XVI | HPC——4 Citric Acid-75 Aerosil——21 | 51 | 22 | Eud.E-50 CaP—50 | 70 | 4-4.5 | 60-74 | 80-100 | 1.5-3 |
| | | | | | 77 | 7-6 | 11-9 | 48-36 | 4-3 |
| | | | | | 85 | 10 | * | 97-85 | 2-1 |
| XVII | HPC——4 Citric Acid-75 Aerosil——21 | 72 | 32 | Eud.E-50 CaP—50 | 61 | 2 | 93 | 100 | 1 |
| | | | | | 75 | 3 | 100 | 100 | 0.5 |
| | | | | | 97 | 3.5 | 100 | 100 | 0.5 |
| | | | | | 88 | 2.5 | 68 | 68-85 | 2.5 |
| XVIII | HPC——4 Citric Acid-75 Aerosil——21 | 71 | 31 | Eud.E-30 CaP—70 | 111 | 4-3.5 | 73-79 | 100 | 1 |

* Release was checked only after 2 hours.

The results show again that the inner coat containing citric acid is involved in the destruction of the outer coat and consequently results in a burst release.

The outer coat should provide protection such that the capsules remain closed for a predetermined period of time (for a desired lag time) and then release the active ingredient in a "burst" manner. Two formulations of outer coating were checked:

- Eudragit-E/Ca Pectinate ratio: 7:3
- Eudragit-E/Ca Pectinate ratio: 1:1

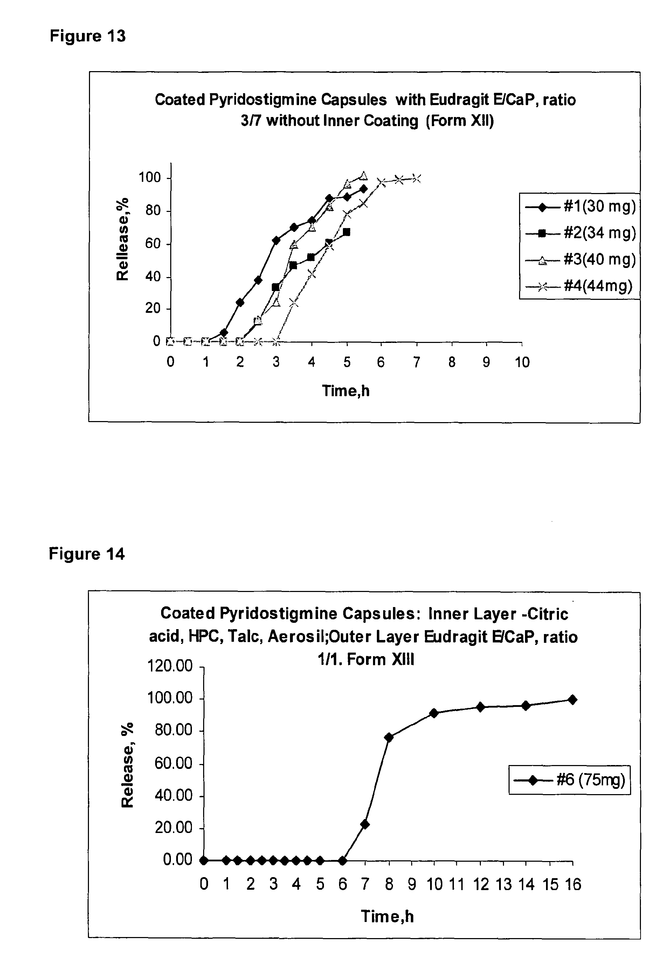

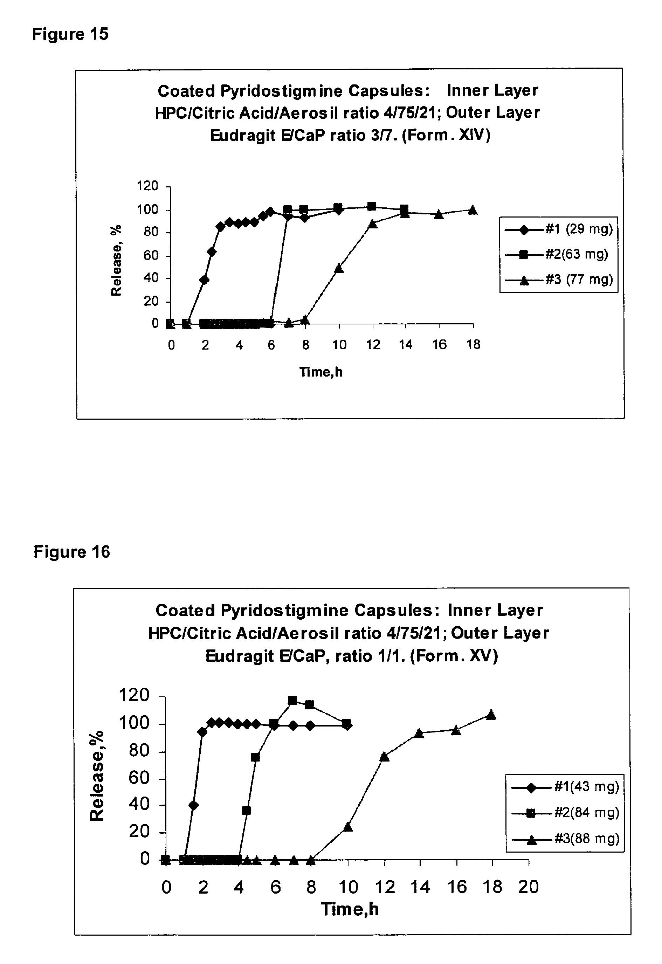

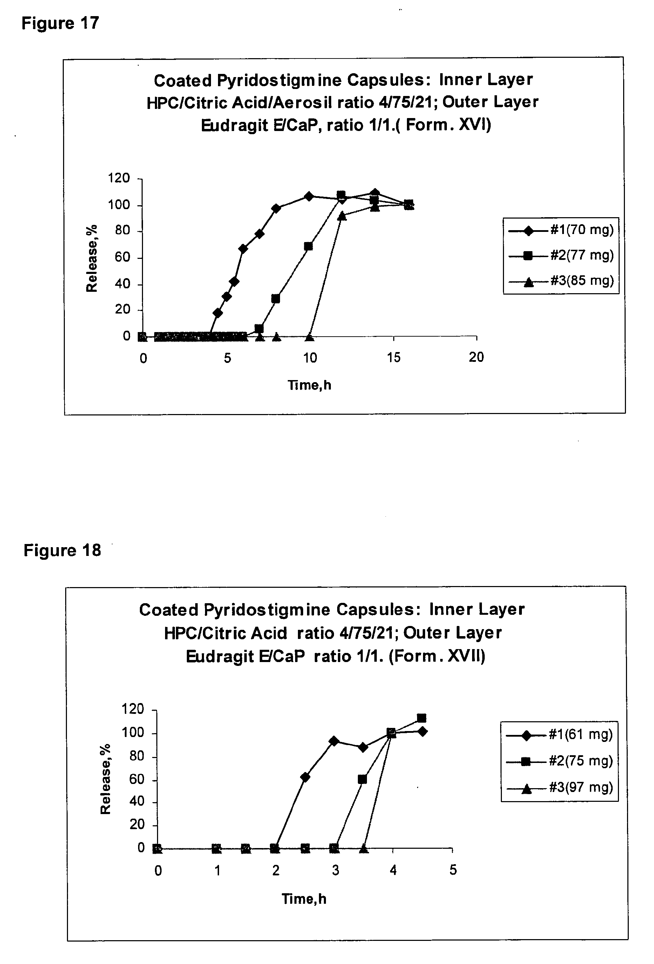

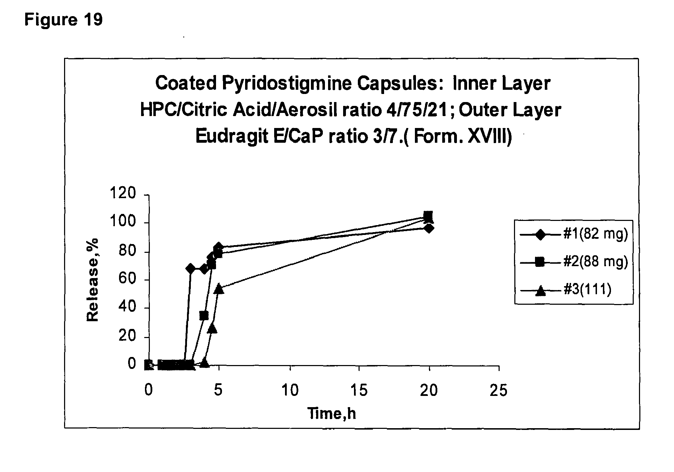

As one can see the formulations XV, XVII, XVIII (with Eudragit-E/Ca Pectinate ratio: 1:1), resulted in the fastest release after the burst of the outer coating took place. The lag time could be controlled by adjusting the outer coating weight. As one could expect the thicker the outer coat, the longer the lag time that may be obtained. On the other hand when the inner layer is thicker, the lag time is shorter for the same outer coat weight (see formulation XVII as compared to XVI). See Figures 13-19, for all formulations related to pyridostigmine coated capsules.

Furthermore, in attempt to determine the weight ratios of the outer coat /inner layer and outer coat/citric acid, required for the desired dissolution, correlation curves were established. Linear correlation curves were drawn between 1. lag time and either weight ratio of outer coat to the inner layer or the weight ratio of outer layer to citric acid included into the inner layer, 2. either weight ratio of outer coat to the inner layer or the weight ratio of outer layer to citric acid included into the inner layer and burst time and burst time (the time takes to 80% of release).

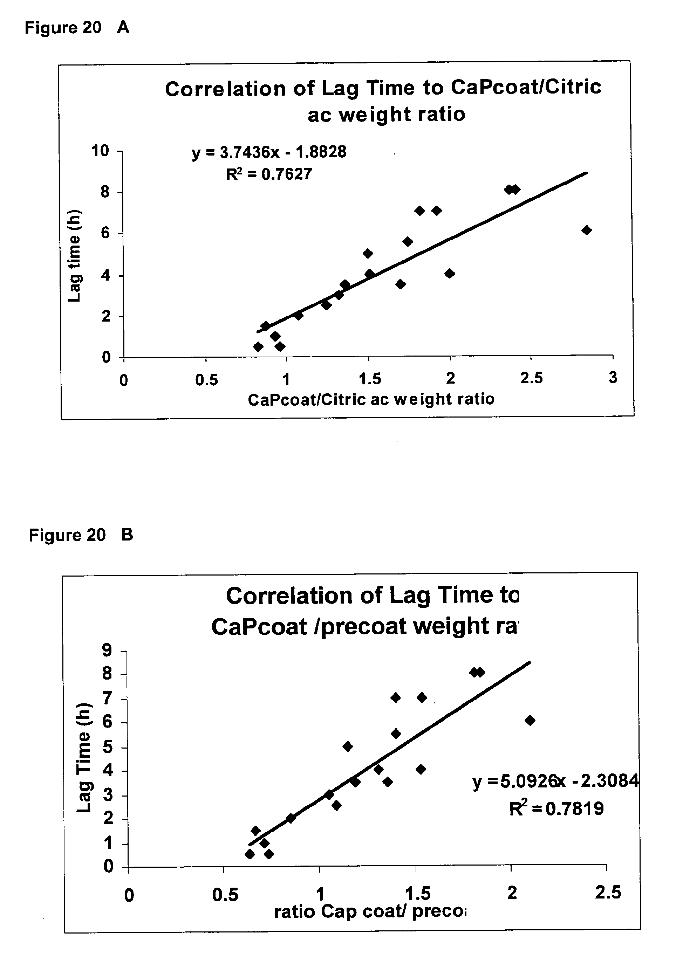

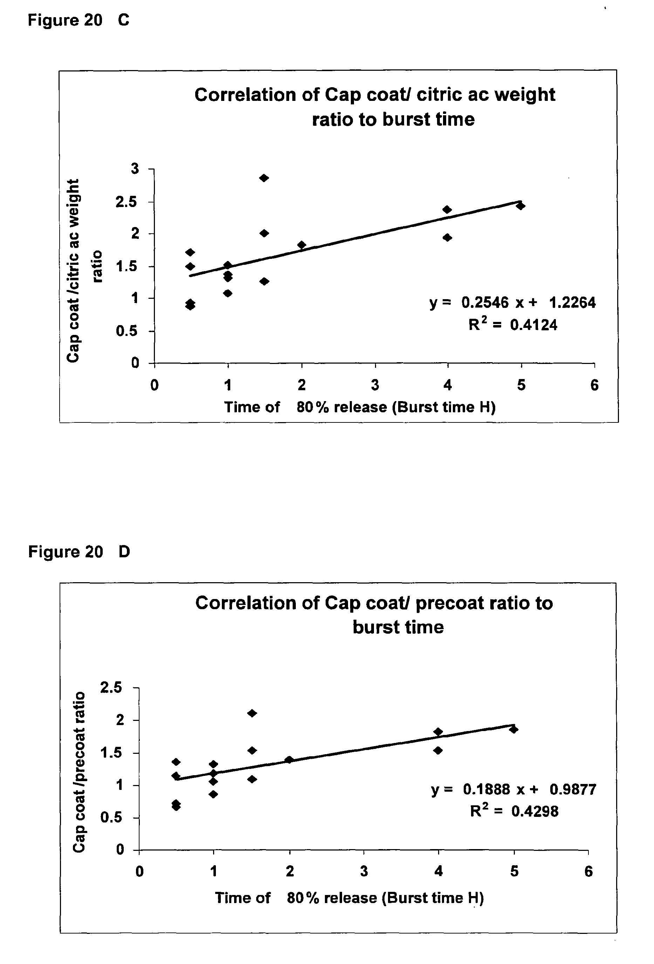

Accordingly, one can find from the linear fit equations, that in order to reach, for example the lag time of 3.5-4.5 hours and a burst time of 0.5-1.5 hours, the weight ratio of outer coat/ inner coat should be 1.0-1.3 and the weight ratio of outer coat/citric acid should be 1.4-1.7 (see Figure 20).

**Claims**

1. A delivery device for the delayed release of an active agent in the gastrointestinal tract comprising:

    a) a core, comprising an active agent;
    b) a first outer coating, comprising a relatively hydrophobic substantially water insoluble polymer having substantially water insoluble hydrophilic particles embedded therein; and
    c) a first inner coating layer, comprising an agent that can cause the dissolution of at least one of said water insoluble components of said outer coating, and optionally a water soluble polymer, such that said insoluble particles in said outer coating, upon absorption of liquid, form channels leading to said inner coating layer, thus enabling the dissolution thereof, whereby the agents contained therein are released to cause at least the dissolution, degradation or destruction of said outer coating, and the release of the active agent from the core of said device.

2. The device of claim 1 for the delayed release of a physiologically acceptable active agent in the gastro-intestinal tract having a physiologically acceptable active agent incorporated therein.

3. The device of claim 1 for the delayed release of a pharmaceutically acceptable active agent in the gastro-intestinal tract having a pharmaceutically acceptable active agent incorporated therein.

4. The device of claim 1 wherein it is further coated with an enteric coating.

5. The device according to claim 4 wherein the enteric coating is selected from the group consisting of hydroxypropylmethyl cellulose phthalate, polyvinyl acetate phthalate, cellulose acetate phthalate, hydroxypropylmethyl cellulose acetate succinate, poly(methacrylic acid, methyl methacrylate)1:1, poly(methacrylic acid, ethyl acrylate)1:1, alginic acid, and sodium alginate.

6. The device according to claim 4 wherein the outer enteric coating further comprises a plasticizer.

7. The device according to claim 6 wherein the plasticizer includes at least one of dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, butyl and/or glycol esters of fatty acids, refined mineral oils, oleic acid, castor oil, corn oil, camphor, glycerol and sorbitol or a combination thereof.

8. The device of claim 1 wherein the relatively hydrophobic polymer of the first outer coating is selected from the group consisting of dimethylaminoethylacrylate/ ethylmethacrylate copolymer, the copolymer being based on acrylic and methacrylic acid esters with a low content of quaternary ammonium groups, wherein the molar ratio of the ammonium groups to the remaining neutral (meth)acrylic acid esters is approximately 1:20, said polymer corresponding to USP/NF "Ammonio Methacrylate Copolymer Type A", an ethylmethacrylate/chlorotrimethylammoniumethyl methacrylate copolymer, the copolymer based on acrylic and methacrylic acid esters with a low content of quaternary ammonium groups wherein the molar ratio of the ammonium groups to the remaining neutral (meth)acrylic acid esters is 1:40, the polymer corresponding to USP/NF "Ammonio Methacrylate Copolymer Type B", a dimethylaminoethylmethacrylate/methylmethacrylate and butylmethacrylate copolymer, a copolymer based on neutral methacrylic acid esters and dimethylaminoethyl methacrylate esters wherein the polymer is cationic in the presence of acids, an ethylacrylate and methylacrylate/ethylmethacrylate and methyl methylacrylate copolymer, the copolymer being a neutral copolymer based on neutral methacrylic acid and acrylic acid esters, ethylcellulose, shellac, and waxes.

9. The device of claim 8 wherein the relatively hydrophobic polymer is ethylcellulose, Eudragit E, Eudragit RL, Eudragit RS, and Eudragit NE.

10. The device of claim 1 wherein the substantially water insoluble hydrophilic particles are selected from the group consisting of water insoluble cross-linked polysaccharide, a water insoluble cross-linked protein, a water insoluble cross-linked peptide, water insoluble cross-linked gelatin, water insoluble cross-linked hydrolyzed gelatin, water insoluble cross-linked collagen, water insoluble cross linked polyacrylic acid, water insoluble cross-linked cellulose derivatives, water insoluble cross-linked polyvinyl pyrrolidone, micro crystalline cellulose, insoluble starch, micro crystalline starch and a combination thereof.

11. The device of claim 10 wherein the water insoluble cross-linked polysaccharide is selected from the group consisting of insoluble metal salts or cross-linked derivatives of alginate, pectin, xantham gum, guar gum, tragacanth gum, locust bean gum, carrageenan, modified cellulose and covalently cross-linked derivatives thereof.

12. The device of claim 10 wherein the substantially water insoluble hydrophilic particles are chosen from insoluble metal salts of a polysaccharide.

13. The device of claim 12 wherein the insoluble metal salts of said polysaccharide are chosen from the group consisting of calcium pectinate and calcium alginate.

14. The device of claim 10 wherein the water insoluble cross-linked cellulose derivatives are selected from the group consisting of cross-linked derivatives of hydroxypropylcellulose, hydroxypropylmethylcellulose, hydroxyethylcellulose, methylcellulose, carboxymethylcellulose, and metal salts of carboxymethylcellulose.

15. The device of claim 10 wherein the substantially water insoluble hydrophilic particles are microcrystalline cellulose.

16. The device according to claim 1 wherein said first inner coating layer comprises a pharmaceutically acceptable water soluble polymer.

17. A device according to claim 16 wherein said water soluble polymer is selected from the group consisting of Povidone (PVP: polyvinyl pyrrolidone), polyvinyl alcohol, copolymer of PVP and polyvinyl acetate, HPC (hydroxypropyl cellulose) (more preferably a low molecular weight), HPMC (hydroxypropyl methylcellulose) (more preferably a low molecular weight), carboxy methyl cellulose (more preferably a low molecular weight), ethylcellulose, hydroxyethyl cellulose, gelatin, polyethylene oxide, acacia, dextrin, magnesium aluminum silicate, starch, polyacrylic acid, polyhydroxyethylmethacrylate (PHEMA), polymethacrylates and their copolymers, gum, water soluble gum, polysaccharide, hydroxypropylmethyl cellulose phthalate, polyvinyl acetate phthalate, cellulose acetate phthalate, hydroxypropylmethyl cellulose acetate succinate, poly(methacrylic acid, methyl methacrylate)1:1 and poly(methacrylic acid, ethyl acrylate)1:1, alginic acid, and sodium alginate, and a mixture thereof.

18. A device according to claim 16 wherein said water soluble polymer is a pharmaceutically acceptable polymer that dissolves in aqueous medium with pH > 4 .

19. A device according to claim 1 wherein said dissolution agent of said first inner coating layer is a non-volatile organic acid.

20. A device according to claim 19 wherein said non-volatile organic acid selected from the group consisting of citric acid, fumaric acid, malic acid, ascorbic acid (Vitamin C), lactic acid, oxalic acid, maleic acid, malonic acid, glutaric acid, adipic acid, pimelic acid, suberic acid, azelaic acid, sebasic acid, tartaric acid, acetic acid, propionic acid, glycolic acid, pyruvic acid, oxalic acid, succinic acid, benzoic acid, cinnamic acid, mandelic acid, methanesulfonic acid, ethanesulfonic acid, p-toluenesulfonic acid, salicylic acid, palmatic acid, and the like, and amino acids selected from the group consisting of aspartic acid and glutamic acid, and a mixture thereof.

21. A device according to claim 1 wherein said dissolution agent of said first inner coating layer is a chelating agent.

22. A device according to claim 21 wherein said chelating agent is selected from the group consisting but not limited to antioxidants, the mono-, di-, tri- and tetrapotassium salt of ethylenediaminetetraacetic acid (EDTA, edetic acid), mono-, di-, tri-and tetrasodium salt of EDTA, disodium calcium salt of EDTA, other EDTA salts, ethylenediaminetetraacetic acid, fumaric acid, malic acid, oxalic acid, maltol, and salts of any pharmaceutically acceptable organic acid.

23. A device according to claim 1 wherein said first outer coating comprises an insoluble metal salt of a polysaccharide in amount of at least 30 % of said first outer film coating weight.

24. A device according to claim 1 further comprising a further water soluble, inner coating layer which separates between said first inner coating and said core.

25. A device according to claim 24 wherein said water soluble, inner coating layer comprises a buffering agent.

26. A device according to claim 24 wherein said water soluble inner layer coating comprises a water soluble polymer selected from the group listed in claim 17.

27. A device according to claim 1 wherein said core comprising a gelatin capsule.

28. A device according to claim 1, wherein the active agent is selected from the group consisting of compounds that act on: the peripheral nerves, adrenergic receptors, cholinergic receptors, nervous system, skeletal muscles, cardiovascular system, smooth muscles, blood circulatory system, blood coagulation system, synaptic sites, neuroeffector junctional sites, endocrine and hormone systems, immunological system, reproductive system, skeletal systems, autocoid systems, alimentary and excretory systems, inhibitory and histamine systems and the central nervous system.

29. A device according to claim 1, wherein the active agent is selected from the group consisting of anti-hypertensives, diuretics, anti-arrthrytics cholesterol lowering drugs, immunosuppressants, steroidals, anti-inflammatories, hormonals, anti-psoriatics hypoglycemics, analgesics, antiviral drugs, antimicrobials anti-parasitics, anti-cancer drugs, antiepileptics, CNS stimulants, CNS depressants, antidepressants, 5 HT inhibitors, anti-schizophrenics, anti-Alzheimer drugs, anti-ulcer drugs, proton pump inhibitors, anti-asthmatics, anticoagulation drugs and vitamins.

30. A device according to claim 3 wherein said pharmaceutically acceptable active agent is selected from a group of drugs of poor bioavailability, said drugs being selected from the group consisting of anti-hypertensives, immunosuppressants, anti-inflammatories, diuretics, antiepileptics, cholesterol lowering drugs, hormonals hypoglycemics, antiviral drugs, nasal decongestants, antimicrobials, anti-arthritics, analgesics, anti-cancer drugs, anti-parasitics, proteins, peptides, polypeptides, CNS stimulants, CNS depressants, 5 HT inhibitors, anti-schizophrenics, anti-Alzheimer drugs, anti-psoriatics, steroidals, oligonucleotides, anti-ulcer drugs, proton pump inhibitors, anti-asthmatics, thrombolytics and vitamins.

31. A device according to claim 3 wherein said pharmaceutically acceptable active agent is a compound metabolized by cytochrome P450 3A enzymes, said compound being selected from the group consisting of Alprazolam, Amiodarone, Amitriptyline, Astemizole, Atrovastatin, Budesonide, Bupropion, Buspirone, Caffeine, Carbamazepime, Cerivastatin, Cisapride, Claritromycin, Clomipramin, Clonazepam, Codeine, Cyclosporine, Dexametazone, Dextrometorphan, DHEA, Diazepam, Diltiazem, Disopiramide, Donepezil, Doxycicline, Erytromycin, Estradiol, Ethylestradiol, Felodipine, Fluoxetine, Imipramine, Lanzoprazole, Lidocaine, Loratidine, Lovastatin, Midazolam, Nefazodone, Nicardipine, Nifedipine, Nizoldipine, Norethindrone, Omeprazole, Ondansetron, Orphenadrine, Paroxetine, Progesterone, Proafenone, Quethiapine, Quinidine, Rifampin, Sertraline, Sibutramine, Sildenafil, Simvastatin, Tacrolimus, Tamoxifen, Terfenadine, Testosterone, Theophyline, Trazodone, Triazolam, Venlafaxine, Verapamyl, Vinblastine, (R)-Warfarin, Zolpidem.

32. A device according to claim 1, wherein the active agent is an active agent selected from the group consisting of peptides, proteins, polypeptides, oligonucleotides and polysaccharides.

33. A device according to claim 32 wherein said polypeptides are selected from the group consisting of, therapeutical agents, nutritional products, steroids, hormones, insulin, growth hormone (GH), growth hormone releasing hormone (GHRH), epithelial growth factor, vascular endothelial growth and permeability factor (VEGPF), nerve growth factor, cytokines, interleukins, interferons, GMCSF, hormone-like products, neurological factor, neurotropic factor, neurotransmitter, neuromodulator, enzyme, antibody, peptide, proteic fragment, vaccine, adjuvant, an antigene, immune stimulating or inhibiting factor, heomatopoietic factor, anti-cancer product, anti-inflammatory agent, anti-parasitic compound, anti-microbial agent, cell proliferation inhibitor or activator, cell differentiating factor, blood coagulation factor, immunoglobulin, anti-angiogenic product, negative selective markers or "suicide" agent, toxic compound, anti-angiogenic agent, and structurally similar bioactive equivalents thereof.

**Figure 1**

**Coated Diclofenac Tablets: Inner Layer Containing no Citric Acid, HPC/Talc ratio 1/1 ; Outer Layer Eudragit E/CaP ratio 3/7 (Form. I)**

**Figure 2**

**Coated Diclofenac Tablets: Inner Layer HPC/Citric Acid/Talc ratio 1/3/1; Outer Layer Eudragit E/CaP ratio 3/7. (Form. II)**

**Figure 3**

Coated Diclofenac Tablets: Inner Layer HPC/Citric Acid/Talc ratio 1/3/1; Outer Layer Eudragit E/CaP, ratio 1/1. (Form. III)

**Figure 4**

Coated Diclofenac Tablets: Inner Layer HPC/EDTA/Talc ratio 12.9/80.6/20; Outer Layer Eudragit E/CaP ratio 3/7 (Form. IV)

**Figure 5**

Coated Diclofenac Tablets: Inner Layer HPC/EDTA 4Na/Talc ratio 13.3/50/16.7; Outer Layer Eudragit E/CaP ratio 3/7. (Form. V)

Legend: #1 (19 mg); #2 (34 mg); #3( 49 mg). Axes: Release,% vs Time,h

**Figure 6**

Coated Tramadol Tablets with Eudragit E/CaP ratio 3/7 without Inner Coating (Form.VI)

Legend: #1(21 mg); #2(32 mg); #3 (39 mg); #4(50 mg). Axes: Release,% vs Time,h

**Figure 7**

Coated Tramadol
Tablets: Inner Layer HPC/EDTA ratio 1/4; Outer
Layer Eudragit E/CaP ratio 3/7 (Form. VII)

**Figure 8**

Coated Tramadol Tablets: Inner Layer
HPMC/EDTA/PEG 400 ratio 20/78/2; Outer Layer
Eudragit E/CaP ratio 3/7. (Form. VIII)

## Figure 9

Coated Tramadol Tablets: Inner Layer
HPMC/Citric Acid/Talc/PEG 400 ratio
38.5/38.5/19.2/3.8; Outer Layer Eudragit E/Cap
ratio 3/7. (Form. IX)

Legend:
- #1 (26 mg)
- #2 (36 mg)
- #3 (44 mg)
- #4 (57 mg)

## Figure 10

Coated Tramadol Tablets with Different Weight of
Inner Layer:ratio HPC/Citric Acid/Talc/Aerosil 2/6/1/1;
Outer Layer Eudragit E/CaP ratio 3/7 (Form X-A and X-B)

Legend:
- X-A #1 (36 mg)
- X-B #1 (42mg)

**Figure 11**

Coated Tramadol Tablets with Different Weight of Inner Layer ratio HPC/Citric Acid/Talc/Aerosil 2/6/1/1; Outer Layer Eudragit E/CaP ratio 3/7 (Form. X-A and X-B)

Legend: X-A #2(44 mg); X-B #2(50mg)

**Figure 12**

Coated Tramadol Tablets: Inner Layer HPC/Citric Acid/Talc/Aerosil ratio 2/6/1/1; Outer Layer Eudragit E/CaP ratio 1/1 (Form. XI)

Legend: #1 (39 mg); #2 (48mg); #3 (58mg)

**Figure 13**

Coated Pyridostigmine Capsules with Eudragit E/CaP, ratio 3/7 without Inner Coating (Form XII)

**Figure 14**

Coated Pyridostigmine Capsules: Inner Layer -Citric acid, HPC, Talc, Aerosil;Outer Layer Eudragit E/CaP, ratio 1/1. Form XIII

**Figure 15**

Coated Pyridostigmine Capsules:   Inner Layer HPC/Citric Acid/Aerosil ratio 4/75/21; Outer Layer Eudragit E/CaP ratio 3/7. (Form. XIV)

#1 (29 mg)
#2 (63 mg)
#3 (77 mg)

**Figure 16**

Coated Pyridostigmine Capsules: Inner Layer HPC/Citric Acid/Aerosil ratio 4/75/21; Outer Layer Eudragit E/CaP, ratio 1/1. (Form. XV)

#1 (43 mg)
#2 (84 mg)
#3 (88 mg)

**Figure 17**

Coated Pyridostigmine Capsules: Inner Layer
HPC/Citric Acid/Aerosil ratio 4/75/21; Outer Layer
Eudragit E/CaP, ratio 1/1.( Form. XVI)

**Figure 18**

Coated Pyridostigmine Capsules: Inner Layer
HPC/Citric Acid ratio 4/75/21; Outer Layer
Eudragit E/CaP ratio 1/1. (Form. XVII)

**Figure 19**

Coated Pyridostigmine Capsules: Inner Layer
HPC/Citric Acid/Aerosil ratio 4/75/21; Outer Layer
Eudragit E/CaP ratio 3/7.( Form. XVIII)

Figure 20   A

**Correlation of Lag Time to CaPcoat/Citric ac weight ratio**

y = 3.7436x - 1.8828
$R^2$ = 0.7627

Figure 20   B

**Correlation of Lag Time to CaPcoat /precoat weight ra**

y = 5.0926x - 2.3084
$R^2$ = 0.7819

Figure 20  C

## Correlation of Cap coat/ citric ac weight ratio to burst time

y = 0.2546 x + 1.2264
R$^2$ = 0.4124

Cap coat /citric ac weight ratio

Time of  80 % release (Burst time H)

Figure 20  D

## Correlation of Cap coat/ precoat ratio to burst time

y = 0.1888 x + 0.9877
R$^2$ = 0.4298

Cap coat /precoat ratio

Time of  80 % release (Burst time H)

**European Patent Office**

## EUROPEAN SEARCH REPORT

Application Number

EP 06 25 2972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 5 472 710 A (KLOKKERS-BETHKE ET AL) 5 December 1995 (1995-12-05) | 1-3, 8-22, 24-26, 28-31 | INV. A61K9/28 A61K9/20 A61K9/48 |
| Y | * column 5, line 36 - line 45 * | 1-9, 16-20, 23,24, 26-30, 32,33 | |
| | * column 6, line 9 - line 31 * <br> * column 7, line 40 - line 57 * <br> * column 8, line 5 - line 14 * <br> ----- | | |
| Y | US 2002/044975 A1 (WATANABE SHUNSUKE ET AL) 18 April 2002 (2002-04-18) | 1-9, 16-20, 24, 26-30, 32,33 | |
| | * page 2, paragraphs 15,16,19 * <br> * page 3, paragraph 33 * <br> * page 5, paragraph 47-51 * <br> * page 6, paragraph 55 * <br> * page 14 - page 15; example 35 * <br> ----- | | TECHNICAL FIELDS SEARCHED (IPC) A61K |
| Y | EP 0 425 699 A (CHUGAI SEIYAKU KABUSHIKI KAISHA) 8 May 1991 (1991-05-08) <br> * page 3, line 3 - line 58 * <br> * page 4 - page 5; example 1 * <br> * claims 1,2,4,5,8-10,13 * <br> ----- | 4-7 | |
| D,Y | US 6 231 888 B1 (LERNER E. ITZHAK ET AL) 15 May 2001 (2001-05-15) <br> * column 12, line 65 - column 13, line 48 * <br> * claims 1,8,13-15,17,25 * <br> ----- | 23,27 | |
| | -/-- | | |

The present search report has been drawn up for all claims

| Place of search | Date of completion of the search | Examiner |
|---|---|---|
| The Hague | 18 September 2006 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 06 25 2972

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | US 5 593 697 A (BARR ET AL) 14 January 1997 (1997-01-14) * column 7 - column 8; example 2 * * column 3, line 30 - line 54 * * column 4, line 3 - line 26 * * column 5, line 2 - line 65 * * claims 1-5 * | 32,33 | |
| A | US 4 871 549 A (UEDA ET AL) 3 October 1989 (1989-10-03) * column 2, line 38 - column 4, line 9 * * claims 1-6 * * figures 1,2 * | 1-33 | |

TECHNICAL FIELDS SEARCHED (IPC)

The present search report has been drawn up for all claims

| Place of search | Date of completion of the search | Examiner |
|---|---|---|
| The Hague | 18 September 2006 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.                    EP 06 25 2972

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2006

| Patent document cited in search report | | Publication date | Patent family member(s) | | Publication date |
|---|---|---|---|---|---|
| US 5472710 | A | 05-12-1995 | NONE | | |
| US 2002044975 | A1 | 18-04-2002 | NONE | | |
| EP 0425699 | A | 08-05-1991 | AT | 109973 T | 15-09-1994 |
| | | | CA | 2032475 A1 | 12-11-1990 |
| | | | DE | 69011618 D1 | 22-09-1994 |
| | | | DE | 69011618 T2 | 15-12-1994 |
| | | | DK | 425699 T3 | 21-11-1994 |
| | | | ES | 2062527 T3 | 16-12-1994 |
| | | | WO | 9013286 A1 | 15-11-1990 |
| | | | JP | 2967492 B2 | 25-10-1999 |
| | | | JP | 3072417 A | 27-03-1991 |
| US 6231888 | B1 | 15-05-2001 | AT | 291417 T | 15-04-2005 |
| | | | AU | 713722 B2 | 09-12-1999 |
| | | | AU | 1206597 A | 11-08-1997 |
| | | | CN | 1208343 A | 17-02-1999 |
| | | | CZ | 9802198 A3 | 16-12-1998 |
| | | | DE | 69732830 D1 | 28-04-2005 |
| | | | DE | 69732830 T2 | 13-04-2006 |
| | | | EP | 0877604 A1 | 18-11-1998 |
| | | | ES | 2241031 T3 | 16-10-2005 |
| | | | WO | 9725979 A1 | 24-07-1997 |
| | | | IL | 125042 A | 20-03-2005 |
| | | | JP | 2000503316 T | 21-03-2000 |
| | | | NZ | 324808 A | 28-10-1999 |
| | | | US | 5840332 A | 24-11-1998 |
| | | | ZA | 9700405 A | 30-07-1997 |
| US 5593697 | A | 14-01-1997 | AT | 194910 T | 15-08-2000 |
| | | | WO | 9217165 A1 | 15-10-1992 |
| | | | CA | 2106952 A1 | 27-09-1992 |
| | | | DE | 69231291 D1 | 31-08-2000 |
| | | | DE | 69231291 T2 | 18-01-2001 |
| | | | EP | 0576522 A1 | 05-01-1994 |
| | | | ES | 2149174 T3 | 01-11-2000 |
| | | | JP | 6507155 T | 11-08-1994 |
| | | | NZ | 242065 A | 25-06-1996 |
| | | | NZ | 286242 A | 24-11-1997 |
| US 4871549 | A | 03-10-1989 | AU | 592581 B2 | 18-01-1990 |
| | | | AU | 5979186 A | 22-01-1987 |
| | | | CA | 1282698 C | 09-04-1991 |
| | | | DE | 3682135 D1 | 28-11-1991 |
| | | | DK | 344586 A | 20-01-1987 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 06 25 2972

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2006

| Patent document cited in search report | Publication date | Patent family member(s) | Publication date |
|---|---|---|---|
| US 4871549 A | | EP 0210540 A1<br>IE 59236 B1<br>JP 2058495 C<br>JP 7072130 B<br>JP 62030709 A<br>JP 7196477 A | 04-02-1987<br>26-01-1994<br>10-06-1996<br>02-08-1995<br>09-02-1987<br>01-08-1995 |

EPO FORM P0459

For more details about this annex : see Official Journal of the European Patent Office, No. 12/82

46

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4871549 A, Ueda
- WO 9832425 A, Busetti
- US 5788987 A, Busetti
- US 5891474 A, Busetti
- US 5840332 A
- US 5472710 A, Klokkers-Bethke
- WO 9009168 A
- US 5593697 A
- US 5260069 A
- US 5472708 A
- US 5260068 A

**Non-patent literature cited in the description**

- **T. ISHIBASHI et al.** *Journal of Pharmaceutical Sciences,* 1998, vol. 87, 531
- *Proceed. Intern. Symp. Control. Rel. Bioact. Mater.,* 1994, vol. 21, 744
- **GOODMAN ; GILMAN'S.** The Pharmacological Basis of Therapeutics. The Merck Index